# EUROPEAN PATENT APPLICATION

(11) **EP 0 984 065 A1**
(43) Date of publication of application: **08.03.2000**
(21) Application number: 98902762.8
(22) Date of filing: 13.02.1998
(51) Int. Cl.: C12N 15/31, C07K 14/00, C07K 14/20, C07K 16/12, C12P 21/02, A61K 39/00, G01N 33/571

(54) **MODIFIED TREPONEMA PALLIDUM-DERIVED ANTIGEN PROTEIN**

(30) Priority: 14.02.1997 JP 2987797; 14.02.1997 JP 2987897
(71) Applicant: HIGETA SHOYU CO., LTD., Chuo-ku, Tokyo 103 (JP); Hitachi Chemical Company, Ltd., Tokyo 163-0449 (JP)
(72) Inventor: KUROIWA, Yasuyuki, Hitachinaka-shi Ibaraki 312-0062 (JP); OKAZAKI, Nobuyuki, Hitachi-shi Ibaraki 317-0054 (JP); IIJIMA, Hiromi, Hitachi-shi Ibaraki 317-0066 (JP); NAKAO, Yoshiki, Hitachi-shi Ibaraki 317-0076 (JP); SAWAZAKI, Takeshi, Hitachinaka-shi Ibaraki 312-0062 (JP); MIZUKAMI, Makoto, Kashima-gun Ibaraki 314-0040 (JP); NISHIKAWA, Yukihiro, Choshi-shi Chiba 288-0045 (JP)
(74) Representative: Woods, Geoffrey Corlett
(86) International application number: JP9800605
(87) International publication number: WO9836076

(57) **Abstract**

A novel modified protein; a DNA encoding the same; a recombinant vector containing the DNA; a transformant containing the recombinant vector, a process for producing the modified protein; a method for assaying anti-Treponema pallidum antibody; an assay reagent therefor; a diagnostic for syphilis infection; and a process for producing anti-Treponema pallidum antibody.

## Description

### Field of the Invention

The present invention relates to a modified protein, DNA encoding the protein, a recombinant vector containing the DNA; a transformant containing the recombinant vector, a process for producing the modified protein, a method and reagent for assaying anti-*Treponema pallidum* antibody, a diagnostic reagent for syphilis infection, and a process for producing the anti-*Treponema pallidum* antibody.

### Background of the Invention

An organism includes various proteins.

Among all, lipoproteins (i.e., lipid-protein complexes) of various pathogenic microorganisms are known to act as antigenic proteins by remaining on cell membranes. Immunoassays commonly used in a test or diagnosis of infection with these microorganisms utilize, as antigens, fractions comprising lipoprotein derived from those pathogenic microorganisms.

For example, *Treponema pallidum* is known as syphilis pathogenic bacterium. Immunoassays currently used in a test or diagnosis of infection with the bacterium utilize, as antigen, cell membrane fractions derived from the *Treponema pallidum*.

Since *Treponema pallidum* can grow only in cells or tissues of the infected animal, a method has been used which comprises the steps of infecting a rabbit orchis with *Treponema pallidum*, collecting the *Treponema pallidum* cells from the infected orchis, and then obtaining cell membrane fractions from the cells in order to obtain antigenic cell membrane fractions of *Treponema pallidum*. However, a large amount of antigens could not be obtained by this method.

A cell membrane fraction of *Treponema pallidum* contains a variety of antigenic proteins which are lipoproteins. It is known that there exist three antigenic proteins which have a molecular weight of about 47 kDa, about 17 kDa, and about 15 kDa respectively. Amino acid sequences of the antigenic proteins, as well as the nucleotide sequences which encode them, have also been reported. See L. M. Weigel et al., *Infect. Immun*., Vol.60, pp. 1568-1576(1992), D. R. Akins et al., *Infect. Immun*., Vol.61, pp.1202-1210(1993), and B. K. Purecell et al., *Mol. Microbiol*., Vol.4, pp.1371-1379(1990). These literatures disclose that the approximately 47-kDa, 17-kDa and 15-kDa antigenic proteins were respectively expressed as fusion proteins with glutathione-S-transferase using recombinant DNA technology.

However, the purpose to produce a large amount of those antigenic proteins has not been achieved since the antigenic proteins, lipoproteins, are not easily released out of the cells.

Also, in recent years, there has been an increased demand for immunoassays with improved sensitivity to infection with the pathogenic bacterium, but the prior technologies as described above wherein the antigenic proteins were merely expressed alone or as fusion proteins with glutathione-S-transferase could not sufficiently satisfy the above demand.

### Problems to be solved by the Invention

The present invention provides a modified protein which can be secreted by microorganisms, with less protein denaturation and high productivity.

The present invention also provides a modified protein which has a high efficiency of secretion in addition to the above advantages.

The present invention also provides a modified protein with antigenicity as a *Treponema pallidum* antigen in addition to the above advantages.

The present invention also provides a modified protein with antigenicity as a *Treponema pallidum*-specific antigen in addition to the above advantages.

The present invention also provides a modified protein with excellent antigenicity as a *Treponema pallidum*-specific antigen in addition to the above advantages.

The present invention also provides a modified protein which is a fusion protein with excellent antigenicity as an antigen from microorganism in addition to the above advantages.

The present invention also provides a modified protein, which is a fusion protein with excellent antigenicity as a *Treponema pallidum*-specific antigen in addition to the above advantages.

The present invention also provides a modified protein with extremely excellent antigenicity as a *Treponema pallidum*-specific antigen in addition to the above advantages.

The present invention also provides DNA useful for producing a modified protein which can be secreted by microorganisms.

The present invention also provides DNA useful for producing a modified protein with excellent antigenicity as a *Treponema pallidum*-specific antigen in addition to the above advantages.

The present invention also provides a recombinant vector useful for producing the modified protein which can be secreted by microorganisms.

The present invention also provides a transformant useful for producing the modified protein which can be secreted by microorganisms.

The present invention also provides a transformant useful for producing the modified protein with excellent antigenicity as a *Treponema pallidum*-specific antigen in addition to the above advantages.

The present invention also provides a process for producing the modified protein which can be secreted by microorganisms.

The present invention also provides a simple process for producing the modified protein with excellent antigenicity in addition to the above advantages.

The present invention also provides a method for assaying an anti-*Treponema pallidum* antibody which is useful for diagnosis of syphilis infection by using, as an antigen, a modified protein which can be secreted by microorganisms and has antigenicity as a *Treponema pallidum* antigen.

The present invention also provides a reagent for assaying an anti-*Treponema pallidum* antibody useful for diagnosis of syphilis infection, comprising the modified protein which can be secreted by microorganisms and has antigenicity as a *Treponema pallidum* antigen.

The present invention also provides a diagnostic reagent for syphilis infection useful for diagnosis of syphilis infection, comprising, as an active ingredient, the modified protein which can be secreted by microorganisms and has antigenicity as a *Treponema pallidum* antigen.

The present invention also provides a process for producing anti-*Treponema pallidum* antibodies useful for diagnosis of syphilis infection.

### Disclosure of the Invention

The present invention relates to:
(1) a modified protein comprising an amino acid sequence wherein at least one cysteine residue is changed to an amino acid residue to which a lipid cannot bind in comparison with the amino acid sequence of the original protein;
(2) the modified protein of (1) which comprises an amino acid sequence wherein the cysteine residue closest to the amino-terminus is changed to an amino acid residue to which a lipid cannot bind in comparison with the amino acid sequence of the original protein;
(3) the modified protein of (1) or (2) wherein the amino acid residue is selected from a group consisting of alanine, glutamic acid and serine residues;
(4) the modified protein of any one of (1) - (3) wherein the original protein is an antigenic protein derived from *Treponema pallidum*;
(5) the modified protein of (4) wherein the original protein is an antigenic protein from *Treponema pallidum* which has a molecular weight of about 47 kDa or about 17 kDa;
(6) the modified protein of (5) comprising the an amino acid sequence shown as SEQ ID NO: 1;
(7) the modified protein of (5) comprising the amino acid sequence shown as SEQ ID NO: 2;
(8) the modified protein of any one of (1) - (7) which is a fusion protein obtainable by linking two or more antigenic proteins from a microorganism or microorganisms;
(9) the modified protein of (8) wherein said antigenic protein derived from the microorganism(s) is a fusion protein comprising a *Treponema pallidum*-derived antigenic protein which has a molecular weight of about 47kDa and/or a *Treponema pallidum*-derived antigenic protein which has a molecular weight of about 17 kDa;
(10) the modified protein of (8) or (9) comprising the amino acid sequence shown as SEQ ID NO: 4;
(11) DNA encoding the modified protein of any one of (1) - (10), or complementary DNA thereto;
(12) The DNA of (11) comprising the nucleotide sequence shown as SEQ ID NO: 5, 6 or 8;
(13) a recombinant vector containing the DNA of (11) or (12);
(14) a transformant containing the recombinant vector of (13);
(15) the transformant of (14) which has been deposited as FERM BP-5641, FERM BP-5642 or FERM BP-5763;
(16) a process for producing a modified protein, comprising the steps of culturing a transformant into which a gene encoding the modified protein of any one of (1)-(10) is introduced, producing and accumulating the modified protein in the culture, and collecting the culture;
(17) the process for producing a modified protein of (16), further comprising the steps of adding ammonium sulfate to the culture supernatant of the collected culture, centrifuging the mixture to obtain a precipitate, and dissolving the precipitate in an aqueous solution containing a surfactant;
(18) a method for assaying an anti-*Treponema pallidum* antibody wherein the modified protein of any one of (4)-(10) is used as an antigen;
(19) a reagent for assaying an anti-*Treponema pallidum* antibody, comprising the modified protein of any one of (4)-(10) as an antigen;
(20) a diagnostic agent for syphilis infection comprising the modified protein of any one of (4)-(10) as an active ingredient;
(21) a process for producing an anti-*Treponema pallidum* antibody wherein the modified protein of any one of (4)-(10) is used as an antigen;

The present invention will be described in detail below.

In the sequence listing, the amino acid at amino-terminus of each amino acid sequence is designated as the first amino acid while the base at 5'-end of each nucleotide sequence is designated as the first base.

### Modified protein

A modified protein of the present invention comprises an amino acid sequence wherein at least one cysteine residue is changed to an amino acid residue to which a lipid cannot bind in comparison with the amino acid sequence of the original protein (hereinafter referred to as "amino acid sequence (A)").

The proteins used in the present invention include, but are not limited to, for example, those derived from organisms such as animals, plants, microorganisms and viruses.

Examples of such proteins are non-secretory proteins such as membrane proteins and cytoplasmic proteins in view of secretion from cells. The membrane proteins include, for example, various enzymes and receptors present on cell membranes. The non-secretory proteins include lipoproteins, which are lipid-bound proteins.

Proteins such as antigenic proteins present on cell membranes are also preferable in terms of antigenicity.

The antigenic proteins present on cell membranes include those derived from microorganisms such as *Treponema pallidum*, *Chlamydia pneumoniae*, *Chlamydia trachomatis*, various hepatitis viruses, human immunodeficiency virus (HIV), adult leukemia virus, herpes virus, RS virus, rubella virus, varicella virus, mycoplasma and toxoplasma gondii.

The antigenic proteins derived from *Treponema pallidum* include, for example, those having a molecular weight of about 47 kDa, about 17 kDa or about 15 kDa. The antigenic proteins derived from *Chlamydia pneumoniae* include, for example, those having a molecular weight of about 39.5 kDa, about 53 kDa or about 70 kDa. The antigenic proteins derived from *Chlamydia trachomatis* include, for example, those having a molecular weight of about 39.5 kDa, about 59.5 kDa or about 75 kDa. These antigenic proteins may be native proteins (maturation proteins) which those microorganisms inherently have, modified proteins comprising an amino acid sequence wherein at least one amino acid is modified in comparison with the amino acid sequence of the native protein, or fusion proteins obtainable by linking two or more antigenic proteins.

Such antigenic proteins from those microorganisms include one which has at least one cysteine residue to which a lipid binds to form a lipoprotein wherein the produced antigenic protein becomes a cell membrane protein. When the antigenic proteins are produced using recombinant DNA techniques, antigenic proteins produced in cells of recombinant microorganisms or the like may be retained within the recombinant cells and insolubilized to form inclusion bodies within the cells, resulting in possible denaturation or reduced productivity (or yield) of the antigenic proteins.

Therefore, the modified protein of the present invention preferably comprises an amino acid sequence (A) wherein at least one cysteine residue is changed to an amino acid residue to which a lipid cannot bind in comparison with the amino acid sequence of the native protein (or maturation protein), whereby the modified protein can be secreted out of microorganisms.

In terms of lipid-binding, the modified protein preferably comprises the amino acid sequence (A) wherein a cysteine residue at the amino-terminal side is changed to an amino acid residue to which a lipid cannot bind in comparison with the amino acid sequence of the native protein. More preferably, the modified protein comprises the amino acid sequence (A) wherein the cysteine residue closest to the amino-terminus is changed to an amino acid residue to which a lipid cannot bind in comparison with the amino acid sequence of the native protein.

More than one cysteine residues may be changed as long as the resulting modified protein keeps properties of the original protein such as antigenicity as *Treponema pallidum* antigen and is able to be secreted by microorganisms.

The amino acid residues to which a lipid cannot bind are not limited to particular ones so long as they are not cysteine residues which can bind via fatty acids to cell membranes of microorganisms which produce the modified proteins. Examples of the amino acid residue to which a lipid cannot bind include glycine, alanine, valine, isoleucine, leucine, phenylalanine, proline, serine, threonine, aspartic acid, glutamic acid, asparagine, glutamine, histidine, lysine, arginine, tryptophan, and tyrosine. Hydrophilic amino acid residues which have small side chains such as alanine, glutamic acid and serine are preferred in terms of secretion efficiency. More preferable is, for example, alanine due to its unaltered antigenicity.

The amino acid sequence (A) may be one wherein some amino acid residues (for example, 1 to 20 amino acid residues) are deleted in a certain region of the amino acid sequence of an antigenic protein derived from *Treponema pallidum* (for example a region from amino acid 1 to amino acid 50 of the antigenic protein) as long as the resulting modified protein keeps properties of the original protein such as the antigenicity as *Treponema pallidum* antigen.

Alternatively, the modified protein of the present invention may be a fusion protein having a structure wherein one or more amino acid sequences bind to the amino-terminus or carboxyl-terminus of the amino acid sequence (A) directly or via intervening amino acid sequence or sequences as long as the resulting modified protein can keep the above properties and can be secreted by microorganisms without losing its antigenicity. Such proteins include, for example, a fusion protein wherein amino acid sequences of 47 kDa-and 17 kDa-antigens from *Treponema pallidum* are linked together. When the modified protein is a fusion protein, the original antigenic proteins which constitute the fusion protein may be the same or different though preferably at least one of its constitutive antigenic proteins has different antigenicity from those of other antigenic proteins so that the resultant fusion protein may have multi-antigenicity. Using this fusion protein as an antigen has advantages over using mixture of antigenic proteins which have different antigenicities as an antigen. That is, production of the fusion protein is simpler than that of the mixture of antigenic proteins since the former needs only to prepare the fusion protein while the latter need to prepare individually and then mix a plurality of antigenic proteins each having different antigenicity to obtain an antigen having a multi-antigenicity; and thus the fusion protein can be expected to have higher antigenicity than the mixture of antigenic proteins.

The number of antigenic proteins which constitute the fusion protein is not limited to a specific number as long as the resultant fusion protein keeps antigenicity as antigen of microorganism to be used.

Using a fusion protein of antigenic proteins derived from two or more microorganisms as an antigen allows diagnosis to be performed quickly and simply for determining which microorganism or microorganisms have infected the subject since a number of microorganisms can be screened with one antigen.

The length of the intervening amino acid sequence and the type of constitutive amino acids thereof are not limited to particular ones as long as the resulting fusion protein keeps antigenicity as antigen of microorganisms to be used though the intervening amino acid sequence itself may preferably not have any antigenicity. Such intervening amino acid sequences include, for example, isoleucine residues or polyglycine residues which have several to tens of amino acid residues.

Amino acid sequences (A) include those described above.

Further, the amino acid sequence (B) which is recognized as a signal peptide in the microorganism which produces the modified protein may preferably be added to the amino-terminus of amino acid sequence (A) of the modified protein since the resultant modified protein can be easily purified from the culture supernatant of the microorganism which produces the modified protein without a complicated step of purifying the protein- Such amino acid sequences include, for example, an amino acid sequence which is recognized as a signal peptide in, for example, *Bacillus brevis.*

When a modified protein of interest is produced by linking amino acid sequence (A) and amino acid sequence (B), at first a fusion protein comprising the amino acid sequence (A) and the amino acid sequence (B) is produced in a microorganism and transported to the vicinity of the cell membrane of the microorganism. Then the amino acid sequence (A) is cut from the fusion protein by signal peptitase present in the cell membrane and secreted from the microorganism. Thus, the modified protein of interest can be efficiently produced without denaturation which may result from the fact that the protein is insolubilized, forms an inclusion body, and retains in the cell of the microorganism which produces it.

Particular examples of the modified proteins of the present invention may include, for example, those comprising amino acid sequences shown as SEQ ID NO: 1-4.

A protein shown as SEQ ID NO: 1 comprises an amino acid sequence wherein the amino acid residues 1 to 19 are deleted and the cysteine residue at position 20 is replaced by an alanine residue in comparison with the amino acid sequence of an antigenic protein having a molecular weight of about 47 kDa derived from *Treponema pallidum*. The protein has antigenicity as *Treponema pallidum* antigen since it can cause an antigen-antibody reaction with sera from syphilis patients.

A protein shown as SEQ ID NO: 2 comprises an amino acid sequence wherein the amino acid residues 1 to 21 are deleted and the cysteine residue at position 22 is replaced by an alanine residue in comparison with the amino acid sequence of an antigenic protein having a molecular weight of about 17 kDa derived from *Treponema pallidum*. The protein has antigenicity as *Treponema pallidum* antigen since it can cause an antigen-antibody reaction with sera from syphilis patients.

A protein shown as SEQ ID NO: 3 comprises an amino acid sequence wherein the amino acid residues 1 to 17 are deleted and the cysteine residue at position 18 is replaced by an alanine residue in comparison with the amino acid sequence of an antigenic protein having a molecular weight of about 15 kDa derived from *Treponema pallidum*. The protein has the antigenicity as *Treponema pallidum* antigen since it can cause an antigen-antibody reaction with sera from syphilis patients.

A protein shown as SEQ ID NO: 4 is a fusion protein comprising an amino acid sequence wherein the amino acid sequence in which the amino acid residues 1-21 are deleted (i.e., the amino acid sequence starting with the cysteine residue at position 22) in comparison with the amino acid sequence of the antigenic protein having a molecular weight of about 17 kDa derived from *Treponema pallidum* is ligated, via an isoleucine residue, to the carboxyl-terminus of the amino acid sequence in which the amino acid residues 1 to 19 are deleted and the cysteine residue at position 20 is replaced by an alanine residue in comparison with the amino acid sequence of the antigenic protein having a molecular weight of about 47 kDa derived from *Treponema pallidum*. The protein has extremely excellent antigenicity as *Treponema pallidum*-specific antigen since it causes antigen-antibody reaction with sera from syphilis patients, as well as multi-antigenicity such as bi-antigenicity as an antigenic protein having a molecular weight of about 47 kDa and as an antigenic protein having a molecular weight of about 17 kDa both derived from *Treponema pallidum*.

### Process for producing Modified Protein

Chemical synthesis or recombinant DNA technology can be used for preparing a modified protein of the present invention.

Chemical synthesis may include, for example, multiple antigen peptide(MAP) method which is suitable for synthesizing a protein consisting of 30 or less amino acids. A protein is synthesized by using ant commercially available peptide synthesizer. In this method, a protein can be synthesized by repeating the MAP procedure.

One example of recombinant DNA technique comprises the steps of preparing a recombinant vector by introducing DNA encoding a modified protein of the present invention into a vector; inserting the recombinant vector into a host to form a transformant; cultivating the transformant; and utilizing the transformant itself or the culture supernatant thereof.

DNAs encoding modified proteins of the present invention will be described later.

Vectors used in the present invention include, for example, plasmid vectors and phage vectors.

Host cells used in the present invention include, for example, *Bacillus brevis*, *Bacillus subtilis*, *Escherichia coli*, and yeast.

Processes for preparing recombinant vectors and transformants, and processes for producing modified proteins using the transformants will be described below in detail.

The recombinant vectors can be prepared by inserting DNA (which will be described below) encoding a modified protein of the present invention into a vector such as plasmid vector or phage vector which can be replicated in a host cell using any conventional method. Linkers may be used if required. Plasmid vectors may be preferably those which contain drug resistant genes such as erythromycin resistant gene and neomycin resistant gene since transformants to be prepared containing the resultant recombinant vectors can be easily screened and the recombinant vectors may be kept stable in their host cells.

As described below, a plasmid vector used in the present invention should comprise a promoter sequence, Shine-Dalgarno sequence (hereinafter referred to as SD sequence) and a terminator sequence in order to produce the modified proteins of the present invention.

Particular examples of such plasmid vectors include, for example, plasmid pUC118, pUB110, pNU200, pNU210, pHT926, pNH300 and pNH400, among which pUC118 and pUB110 are available from TAKARA Shuzo Co., Ltd. Detailed description of pNU200 is disclosed in "Shigezo Udaka, Journal of the Japan Society for Bioscience, Biotechnology, and Agrochemistry" Vol. 61, p.669 (1987). Detailed description of pNU210 is provided in "Bioindustry, Vol.9, pp.100-107 (1992). Detailed description of pHT926 is disclosed in JP-A-6-133782. Detailed description of pNH300 will be provided in the following examples described later. Detailed description of pNH400 is disclosed in "J. Bacteriol." Vol.177, pp.745-749 (1995).

Phage vectors such as λgt11 phage and λgt10 phage can be used. Recombinant vectors corresponding to the parent vectors used can be obtained using either of them.

When a recombinant DNA technique is used for production of a modified protein of the present invention, recombinant plasmids to be constructed have to be plasmids which can express DNA encoding the protein. Methods for producing such plasmids include, for example, a process comprising the step of inserting DNA of the present invention into a plasmid in the vicinity of and downstream from its promoter sequence and SD sequence, and a process comprising the steps of inserting, in a plasmid, DNA encoding the amino acid sequence (A) into a site downstream from expressable DNA encoding the amino acid sequence (B) in frame (therefore the modified protein of the present invention obtained in the host is a fusion protein comprising the amino acid sequences (A) and (B)). One example of the amino acid sequence (B) may be, for example, a signal peptide such as one from *Bacillus brevis*.

Particular examples of the recombinant plasmid according to the present invention which can express DNA encoding a modified protein of the present invention include, for example, plasmid pNH400TP47, pNH300TP17 and pNH400TP47-17.

A standard procedure for preparing a recombinant plasmid is disclosed in J.Samblook et al., *Molecular Cloning* 2nd ed., Cold Spring Harbor Laboratory Press, 1989 (hereinafter referred to as "Molecular Cloning").

Transformants of the present invention can be prepared by introducing the recombinant plasmids described above into host cells.

Host cells are those capable of expressing DNA of the present invention in the recombinant plasmids, and include, but are not limited to, *Bacillus brevis, Bacillus subtilis*, yeast and *E. coli*. For any of those microorganisms, their strains which do not produce proteolytic enzymes are preferably used in that the modified proteins of the present invention produced in the transformants is hardly decomposed. When the above plasmids pHN400TP47, pNH300TP17 and pNH400TP47-17 are used as recombinant plasmids, *Bacillus brevis* strains such as *Bacillus brevis* 47 strain and *Bacillus brevis* H102 strain (which is the same strain as *Bacillus brevis* HPD31 strain) may be used.

Methods for introducing a recombinant plasmid into a host include, for example, electroporation, protoplast method, calcium chloride method and Tris-PEG method. The method to be selected may depend on the type of the host to be used. For example, when *Bacillus* *brevis* is used as a host, electroporation may preferably be used due to its efficiency and convenience.

A standard method for preparing a transformant using a recombinant plasmid is disclosed in the above "Molecular Cloning". Detailed descriptions of method for producing a transformant using the above *Bacillus brevis* 47 or *Bacillus brevis* H102 as a host is disclosed in, for example, Takahashi et al., *J. Bacteriol.,* Vol.156, pp.1130-1134 (1983); and Takagi et al., *Agric. Biol. Chem.,* Vol.53, pp.3099-3100 (1989).

Particular examples of the transformant of the present invention include, for example, *Bacillus brevis* HPD31 transformed with the above plasmid pNH400TP47 (*Bacillus brevis* HPD31/pNH400TP47), *Bacillus brevis* HPD31 transformed with plasmid pNH300TP17 (*Bacillus brevis* HPD31/pNH300TP17), and *Bacillus brevis* HPD 31 transformed with plasmid pNH400TP47-17 (*Bacillus brevis* HPD31/pNH400TP47-17).

*Bacillus brevis* HPD31/pNH400TP47, *Bacillus brevis* HPD31/pNH300TP17 and *Bacillus brevis* HPD31/pNH400TP47-17 have been deposited with the National Institute of Bioscience and Human-Technology, Agency of Industrial Science and Technology (Japan) as FERM BP-5642, FERM BP-5641 and FERM BP-5763, respectively.

The transformants may be cultured by, for example, inoculating them into a medium in which the transformants can grow and then shaking it or allowing it to stand at an appropriate temperature.

Media containing carbon and nitrogen sources may be used. Carbon sources include, for example, saccharides such as glucose, glycerol, starch, dextran and treacle, and organic acids. Nitrogen sources include, for example, organic nitrogen sources such as casein, peptone, meat extract, yeast extract, casamino acid and glycine, and inorganic nitrogen sources such as ammonium sulfate and ammonium nitrate.

When the transformant shows auxotrophy, trophic substances required for its growth may be added to the medium. Such trohpic substances include, for example, amino acids, vitamins, nucleic acids, and salts.

A synthetic medium primarily containing saccharides and inorganic nitrogen source may be used.

Agar may be added to the medium when a solid medium is used.

Preferably, antibiotics may be added to the medium in order to keep the recombinant plasmids stable and inhibit the growth of strains which do not contain the recombinant plasmids. Such antibiotics include, for example, penicillin, erythromycin, neomycin, chloramphenicol, bacitracin, D-cycloserine and ampicillin.

Antiforming agent such as soybean oil or lard oil and various surfactants may be added to the medium if required.

The medium may be adjusted to a suitable pH which allows the transformants to grow, usually in a range of 5-9, preferably of 6.5-7.5.

One example of media to be used is TMB medium (containing 1% peptone, 0.5% meat extract, 0.2% yeast extract, 1% glucose, 0.01% MgSO₄, 0.01% FeSO₄, 0.001% MnSO₄, 0.0001% ZnSO₄, and 10 µg/ml erythromycin, pH7.0).

Culture temperature may be typically in a range of 15-42°C and preferably 24-37°C when *Bacillus brevis* is used as a host. The culture time depends on various parameters such as type of the host, physical condition of the medium (i.e., solid or liquid), type of the culture device and culture temperature, though the typical culture time is in a range of 16-166 hours, and preferably in a range of 24-96 hours when *Bacillus brevis*, TMB liquid medium and 15-42°C are used as the host, the medium, and the culture temperature, respectively.

One method for producing a modified protein of the present invention using a transformant comprises the steps of culturing a transformant transformed with a gene encoding the modified protein as describe above, producing and accumulating the modified protein in the culture comprising the cultured fluid and the transformant, collecting the culture, and purifying the protein of interest from the culture.

Another method for producing a modified protein comprises the steps of collecting the cultured transformant as the culture, preparing disrupted or lysed cells by disrupting or lysing the culture, and purifying the modified protein of the present invention from the disrupted or lysed cells.

In this embodiment, since the modified protein of the present invention produced in the host is a fusion protein comprising a signal sequence and the above amino acid sequence (A), the signal sequence will be removed from the fusion protein by a signal peptidase of the transformant and the above amino acid sequence (A) will be secreted from the transformant. Therefore, a simple method comprising the steps of collecting the culture supernatant as the culture after culture of the transformant and purifying the modified protein of the present invention from the culture supernatant as amino acid sequence (A) is preferably used.

The yield of the modified protein of interest may be increased using any purification process from the culture fluid and the cultured transformant.

A method for disrupting the transformant may include physically disrupting the transformant by, for example, suspending and sonicating it in a buffer, or by kneading the transformant with quartz sand and suspending the mixture in a buffer. On the other hand, a method for lysing the transformant may include lysing it with, for example, an enzyme for lysing the cell wall of the transformant in combination with, for example, a surfactant. Lysozyme and sodium dodecyl sulfate (SDS) may be used as the enzyme and the surfactant, respectively, when *E. coli* is used as the host of the transformant.

One method for purifying a modified protein of the present invention from the resulting disrupted or lysed cells after disruption or lysis of the transformant may comprise, for example, the steps of centrifuging the above disrupted or lysed cells, removing the resultant cell debris, collecting the supernatant, adding streptomycin sulfate to the supernatant, stirring and centrifuging the mixture to remove nucleic acids as precipitates, collecting the supernatant, stirring the supernatant after adding ammonium sulfate, and centrifuging the mixture to obtain a precipitate. In the last centrifugation, it is preferred to sample the supernatant and precipitate fractions to confirm if the fractions of the supernatant and precipitate contain any modified protein of the present invention since the supernatant may contain the modified protein of the present invention.

One example of the method for purifying a modified protein of the present invention from the culture supernatant after culture of the transformant may comprise the steps of, for example, adding ammonium sulfate (for example 25%) to the culture supernatant, and stirring and centrifuging the mixture to obtain a precipitate. In the method, it is preferred to add ammonium sulfate at a low concentration (for example, 10%) to the culture supernatant at first for removal of contaminants, stir and centrifuge the mixture to obtain the supernatant to which additional ammonium sulfate is added (for example, final concentration of 25%), and then stir and centrifuge the mixture to obtain a precipitate.

The obtained precipitate may preferably be dissolved in an aqueous solution containing a surfactant in order to give a modified protein which has excellent antigenicity. For example, any buffer which is commonly used in protein purification such as PBS or Tris-HCl buffer maybe used as the aqueous solution. Surfactants may include, for example, SDS and Tween 20. The concentration of the surfactant present in the aqueous solution may be preferably in a range of 0.05-2%, more preferably 0.1-1%, and most preferably 0.5-1%. The antigenicity of the resultant modified protein tends to be decreased when the concentration of the surfactant is below 0.05% or over 2%. Particularly, when the protein is derived from *Treponema pallidum,* the resultant precipitate may preferably be dissolved in an aqueous solution containing a surfactant in the above-described concentration in order to give a modified protein which has excellent antigenicity as *Treponema pallidum.*

The modified proteins of the present invention may also be purified by fractionation of the culture supernatant of the transformant using cation exchange chromatography.

Examples of procedures such as removal of cell debris, removal of nucleic acids by adding streptomycin sulfate, and recovery of proteins by adding ammonium sulfate are described in "Molecular Cloning".

### DNA encoding a modified protein

DNA encoding a modified protein herein used refers to DNA selected from a group consisting of DNAs of which nucleotide sequences correspond to the amino acid sequences of the modified proteins based on Genetic Triplet Code (in which 1-6 nucleotide sequences are assigned to each amino acid).

The modified proteins of the present invention include those described in the above section regarding the modified proteins. DNAs encoding the modified proteins include those comprising nucleotide sequences each corresponding to the amino acid sequence of each modified protein.

Examples of the DNA encoding a modified protein include, for example, DNA comprising any one of nucleotide sequences shown as SEQ ID NOS: 5-8.

DNA comprising the nucleotide sequence of SEQ ID NO: 5 encodes the protein comprising the amino acid sequence shown as SEQ ID NO: 1. Segment from position 3 to position 1276 of the nucleotide sequence corresponds to the coding region.

DNA comprising the nucleotide sequence of SEQ ID NO: 6 encodes the protein comprising the amino acid sequence shown as SEQ ID NO: 2. Segment from position 3 to position 407 of the nucleotide sequence corresponds to the coding region.

DNA comprising the nucleotide sequence of SEQ ID NO: 7 encodes the protein comprising the amino acid sequence shown as SEQ ID NO: 3. The entire nucleotide sequence corresponds to the coding region.

DNA comprising the nucleotide sequence of SEQ ID NO: 8 encodes the protein comprising the amino acid sequence shown as SEQ ID NO: 4. Segment from position 3 to position 1655 of the nucleotide sequence corresponds to the coding region.

DNA encoding the modified protein can be prepared using chemical synthesis or recombinant DNA technique.

Chemical synthesis may include, for example, phosphoramidite method which is suitable for synthesizing DNA having a total length of 100 or less base pairs. DNAs can be synthesized by using any commercially available synthesizer.

Not only recombinant DNA technique (which will be described later) but also the following procedure can be used to prepare DNA having a full length of more than 100 base pairs.

A nucleotide sequence is divided into fragments having less than 100 base pairs. The resulting DNA fragments having respective nucleotide sequences are chemically synthesized as described above. Those DNA fragments are then mixed and ligated using T4-DNA ligase. In these steps, DNA of interest may more easily be obtained by synthesizing complementary DNA thereto so that protruding ends are formed to serve as cohesive ends when the corresponding DNA fragments are paired.

One example of the recombinant DNA technique may include performing polymerase chain reaction (PCR) method which utilizes primer DNA which is designed based on the nucleotide sequence of the original protein using, as the template, the genomic DNA (cDNA when the microorganism is eukaryotic) of the organism which produces the original protein.

When *Treponema pallidum* is used as the microorganism which produces the original protein, *Treponema pallidum* Nichols strain (available from Lee Laboratories, U.S.A.) may be used.

Procedure to be used for obtaining genomic DNA or cDNA from the microorganism which produces the original protein is not limited to particular ones and depends on the type of the microorganism. See, for example, processes described in "Molecular Cloning".

Genomic DNA of *Treponema pallidum* can be obtained, for example, by suspending *Treponema pallidum* cells in an aqueous solution containing 1M sodium chloride, 1N sodium hydroxide and 2% SDS, boiling the suspension, adding 0.5M Tris (pH7.0) to neutralize it, extracting with phenol, precipitating with ethanol, and drying the resulting precipitate to obtain a product. Such a process for obtaining genomic DNA is disclosed in detail in M.N. Norgard et al., *J. Clin. Microbiol.*, Vol.29, pp.62-69 (1991).

The nucleotide sequences of antigenic proteins derived from *Treponema pallidum* are disclosed in, for example, L. M. Weigel et al., *supra*, D. R. Akins et al., *supra*, and B. K. Purecell et al., *supra* as describe above. Although a nucleotide sequence of a primer DNA to be used for PCR method may be designed based on those nucleotide sequences of the antigenic proteins, the nucleotide sequence of the primer DNA should be designed to correspond not to the amino acid sequence of the antigenic protein but rather to that of the modified antigenic protein wherein the cysteine residue at the amino-terminus is changed to an amino acid residue to which a lipid cannot bind (such as alanine, glutamic acid or serine residue) in comparison with the amino acid sequence of the native protein. The primer DNA so designed may be produced by chemical synthesis using any commercially available DNA synthesizer.

Common procedures for replicating DNA using PCR are disclosed in "Molecular cloning".

Once DNA encoding the modified protein is obtained, the DNA can be replicated using any recombinant DNA technique or the above PCR method. Thus, the step to obtain the genomic DNA or cDNA of the microorganism which produces the original protein can be omitted.

The modified protein of the present invention can be used as an active ingredient of various diagnostic agents. Particularly, when a protein derived from *Treponema pallidum* is used, the resulting modified protein can be used as an active ingredient of diagnostic agent for detecting syphilis infection since it has antigenicity as *Treponema pallidum*.

### Method and reagent for assaying an anti-Treponema pallidum antibody using the modified protein as an antigen and diagnostic reagent for syphilis infection comprising the modified protein as an active ingredient

A method for assaying an anti-*Treponema pallidum* antibody is not limited to a particular embodiment as long as it utilizes the modified protein of the present invention derived from *Treponema pallidum* as a portion or the whole of the antigen. The modified proteins of the present invention can be used alone or as a mixture thereof.

Methods for assaying an anti-*Treponema pallidum* antibody such as immunoassay using any markers, latex agglutination method using latex carrier particles or immuno nephelometry may be used.

An immunoassay using various markers will be explained in detail below.

One example of assay of anti-*Treponema pallidum* antibody by immunoassay using various markers includes the following steps: preparing an immobilized antigen by physically or chemically immobilizing the above modified protein to a carrier; contacting and incubating the immobilized antigen with a sample for a certain period thereby allowing, if any, the anti-*Treponema pallidum* antibody present in the sample to bind to the above immobilized antigen to form an antigen-antibody complex on the carrier; washing the carrier if necessary; and contacting the antigen-antibody complex with a labeled antibody directed to the above antibody in the sample (if needed, the sample and the labeled antibody may be simultaneously contacted).

Once the above antigen-antibody complex is formed, the labeled antibody will bind to the complex whereby the labeled antibody will also be immobilized onto the carrier. Then, the amount of the label on the labeled antibody which has bound or has not bound to the carrier may be determined by a suitable method for the label used. The obtained value can be used to determine the presence or the amount of the anti-*Treponema pallidum* antibody in the sample.

"Assays" used herein refers not only to quantitative or semi-quantitative assay but also qualitative assay such as detection.

Carriers to be used in the present invention include, but are not limited to, plastic materials such as polystyrene and vinyl chloride, fibrous materials such as cellulose, nitrocellulose and nylon, non-organic materials such as glass and silica gel, erythrocyte, liposome and polyvinylidene difuloride (PDVF) as long as they can immobilize an antigen on themselves. These materials may be in any form such as a microtiter plate, beads, magnetic beads, a paper disc, a membrane and a fiber. Particularly, the carrier may be preferably used in the forms of polystyrene beads or microtiter plate, and more preferably a polystyrene microtiter plate due to convenience.

One example of the method for physically immobilizing the above modified protein on the carrier may include the steps of contacting a solution containing the above modified protein (antigen solution) with the carrier and then allowing the solution to stand at a low temperature (for example, 4°C) overnight.

One example of the method for chemically immobilizing the above modified protein on the carrier may include the steps of mixing the above modified protein, a carrier having carboxyl group on its surface and carbodiimide and allowing the mixture to stand.

Samples (or specimens) may include, for example, various human body fluids. Particularly, human blood, human tear, humor wiped from human pharynx or human urine is preferably used as sample since diagnostic results can be reflected in the sample donor. Human blood is more preferable, human serum is particularly preferable.

Preferably, the surface of the carrier may be blocked with bovine serum albumin or the like before adding the sample in order to prevent non-specific binding of other antibodies or the like present in the sample to the carrier.

Tris or phosphate buffer containing a surfactant, for example, may be used as a washing solution.

The labeled antibodies include antibodies labeled with various labels directed to the antibodies present in the sample (for example, human antibody). Antibodies to be labeled may include anti-human IgG anbtibodies, anti-human IgA antibodies, anti-human IgM antibodies or fragments thereof such as F(ab')₂ and Fab. Labeled antibody to be used may depend on the type of antibodies to be assayed.

Assay may preferably be performed by reacting different types of labeled antibodies individually with one sample in the step of contacting the labeled antibody directed to the antibody present in the sample since diagnostic results can be reflected in the sample donor. The different types of antibodies include labeled anti-human IgG antibodies, labeled anti-human IgA antibodies, and labeled anti-human IgM antibodies.

Labels to be used for labeling an antibody include enzymes, radioisotopes, fluorescent materials and luminescent materials. The enzymes include malate dehydrogenase (EC 1.1.1.37), glucose-6-phosphate dehydrogenase (EC 1.1.1.49), glucose oxidase (EC 1.1.3.4), horseradish peroxidase (EC 1.11.1.7), acetylcholine esterase (EC 3.1.1.7), alkaline phosphatase (EC 3.1.3.1), glucoamylase (EC 3.2.1.3), lysozyme (EC 3.2.1.17), and β-galactosidase (EC 3.2.1.23). Fluorescent materials to be used in the present invention include fluorescein.

Among those labels, enzyme may be preferably used due to its high sensitivity, safety, and convenience. Immunoassay using an enzyme as a label is commonly referred to as enzyme-linked immunosorbent assay (ELISA).

As an enzyme-labeled antibody, alkaline phosphatase- labeled antibody or horseradish peroxidase-labeled antibody is preferably used (both of which are commercially available) since they allow a simple and sensitive assay.

Chemical material such as biotin, avidin, streptoavidin or digoxigenin may intervene between the antibody and the label in order to bind the two.

One example of the method for assaying an anti-*Treponema pallidum* antibody by using latex agglutination method and using the above modified protein may include the steps of preparing an immobilized antigen by physically or chemically immobilizing the above modified protein onto latex particles (carrier), contacting and incubating the immobilized antigen with the sample for a certain period of time, and determining the turbidity of the mixture solution of the immobilized antigen and the sample. Contacting the immobilized antigen and the sample allows, if any, the anti-*Treponema pallidum* antibody present in the sample to bind to the immobilized antigen to form an antigen-antibody complex. During the binding step, due to the existence of two sites in an antibody where it binds to an antigen, a structure is formed where the antigen-antibody complexes are crosslinked each other, causing agglutination. Since the agglutination increases the turbidity of the mixture solution of the sample and the immobilized antigen, the turbidity may be determined by visual observation or using a spectrophotometer.

A modified latex agglutination method may include use of other microparticles such as erythrocyte and liposome instead of latex particles.

The modified proteins of the present invention derived from *Treponema pallidum* can be used as antigens in a reagent for assaying anti-*Treponema pallidum* antibodies.

The components of a reagent for assaying may depend on a method for assaying to be used. One example of the reagent for assaying anti-*Treponema pallidum* antibodies used in immunoassay using the above labels may comprise an immobilized antigen on a carrier and a labeled antibody which reacts with the antibody to be assayed, separately. Reagents for assaying anti-*Treponema pallidum* antibodies used in the above latex agglutination method include those comprising an immobilized antigen on latex particles (carrier).

The above described carriers and labeled antibodies may be used.

The labeled antibodies may be pre-dispersed in, for example, a buffer.

Any labeled human Ig antibody may be used as a labeled antibody in the above reagent. Those labeled human Ig antibodies include, for example, labeled human IgG, IgA and IgM antibodies. Although only one of these antibodies can be used as a labeled antibody for one sample, the three labeled antibodies may preferably be used for one sample since diagnostic results can be reflected in the sample donor. The labeled antibodies may be prepared separately or as a mixture.

The above reagents may optionally be used in combination with other components. For example, in immunoassay using the above labels, components other than the antibody in the reagent include, for example, a negative or positive control sample, a detergent, a reaction substrate (when an enzyme is used as a label), a diluent, a sensitizer, and a reaction stopping solution, which may be used independently or in combination. Those reagents may be used in a various forms such as a kit including suitable amounts of the above components, or a bulk of one of those components.

In latex agglutination method, components other than the antibody in the reagent include, for example, a negative or positive control sample, a detergent, and a sensitizer such as bovine serum albumin or polyethylene glycol.

The above reagents for assaying anti-*Treponema pallidum* antibodies are useful for diagnosis of syphilis infection.

The above reagent comprising the above modified protein as active ingredient *per se* may be used as a diagnostic reagent for syphilis infection.

### Process for producing anti-Treponema pallidum antibodies using the modified proteins as antigens

An anti-*Treponema pallidum* antibody can be obtained as an antiserum or an isolated anti-*Treponema pallidum* antibody using the modified protein of the present invention derived from *Treponema pallidum* as the antigen.

Antiserum may be prepared, for example, by immunizing an animal such as a rabbit or a mouse with the above antigen and obtaining serum from the immunized animal.

Anti-*Treponema pallidum* antibody can be isolated by, for example, immunizing an animal such as a rabbit or a mouse with the above antigen, producing hybridomas by fusing the spleen cells of the animal with myeloma cells, selecting a hybridoma which recognizes the above antigen, culturing the hybridoma, and collecting the resulting culture supernatant. Immunogen may be prepared by optionally binding any suitable carrier protein to the above antigen in order to obtain higher antigenicity.

Freund's complete adjuvant (FCA) and Freund's incomplete adjuvant (FIA) can be preferably used though any adjuvant may be used for immunization.

For example, P3X63Ag8.653 (ATCC (American Type Culture Collection) CRL-1580) or P3/NSI/1-Ag4-1 (ATCC TIB-18) may be used as the myeloma cell. Unlimiting animals such as rabbit, mouse, rat, bovine, sheep, goat or chicken may be used to be immunized with the antigens.

Anti-*Treponema pallidum* antibodies may be produced in accordance with a well known standard procedure for obtaining an antibody by immunizing an animal except using the modified protein of the present invention derived from *Treponema pallidum* as the antigen. Either polyclonal or monoclonal antibodies may be used.

Those antisera and isolated anti-*Treponema pallidum* antibodies may be modified with various enzymes or colloids.

The resulting antisera or isolated anti-*Treponema pallidum* antibodies may be useful for diagnosis of syphilis infection.

### Brief Description of the Drawings

Fig. 1 shows the construction of plasmid pNH400TP47.
Fig. 2 shows the result of sodium dodecyl sulfate-polyacrylamide gel electrophoresis and Western blotting of the modified protein prepared in Example 1.
Fig. 3 shows the construction of plasmid pNH300TP17.
Fig. 4 shows the result of sodium dodecyl sulfate-polyacrylamide gel electrophoresis and Western blotting of the modified protein prepared in Example 2.
Fig. 5 shows the construction of plasmid pNH400TP47-17.
Fig. 6 shows the result of sodium dodecyl sulfate-polyacrylamide gel electrophoresis and Western blotting of the modified protein prepared in Example 4.

### Examples

The present invention will be described in detail by examples below.

### Example 1

Production of a modified protein of the antigenic protein having a molecular weight of about 47 kDa derived from *Treponema pallidum*, examination of antigenicity of the modified protein by Western Blotting, and assay of anti-*Treponema pallidum* antibody by ELISA

### (1) Preparation of recombinant plasmids which express a modified protein of the antigenic protein having a molecular weight of about 47 kDa derived from Treponema pallidum

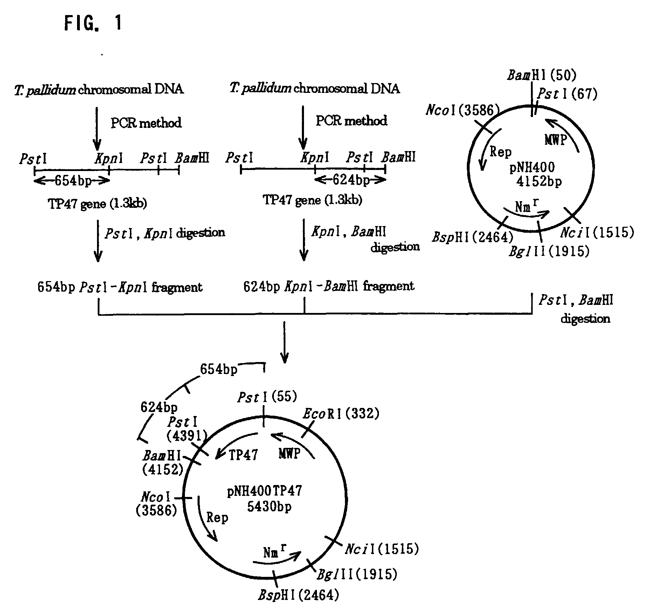

The recombinant plasmids were prepared according to the procedure shown in Fig. 1.

*Treponema pallidum* Nichols strain (available from Lee Laboratories, U.S.A., Product code: SPROJ-TPURE) was used as *Treponema pallidum.*

Genomic DNA from the *Treponema pallidum* was obtained by the method of M.V. Norgard et al. (*supra*) as follows.

*Treponema pallidum* cells (5×10⁷) were suspended in an aqueous solution of 1M sodium chloride, 1N sodium hydroxide and 2% SDS and boiled for one minute. Then, the suspension was neutralized by adding 0.5M Tris (pH7.0) in amount of four times the volume of the solution. Phenol was then added to obtain an aqueous phase to which ethanol was added. Precipitate was collected by centrifugation and dried. Finally, the precipitate was dissolved in sterilized water, which was used as a genomic DNA solution of *Treponema pallidum*. The genomic DNA solution was used as the template DNA in the PCR method which will be described below.

L.M. Weigel et al., *supra*, discloses a nucleotide sequence of DNA encoding an antigenic protein having a molecular weight of about 47 kDa derived from *Treponema pallidum* (shown in SEQ ID NO:9).

The antigenic protein having a molecular weight of 47 kDa is assumed to become a cell membrane protein by a mechanism described below. First, a precursor protein comprising a signal sequence (the 1-434 amino acid sequence shown in combination with the nucleotide sequence in SEQ ID NO: 9) is synthesized in a *Treponema pallidum* cell. Then, a fatty acid present in the cell binds to the cysteine residue at position 20 of the precursor protein via a thioester linkage. The 1-19 amino acid sequence of the precursor protein is removed as a signal sequence by a signal peptidase present in the cell, and the bound fatty acid is fixed in the lipid bilayer of the cell membrane, thereby immobilizing the amino acid sequence starting with amino acid 20 of the precursor protein (maturation protein) as a cell membrane protein.

Since the fatty acid seems to inhibit the expression/secretion of the antigenic protein, a primer DNA for the amino-terminal side was designed so that the cysteine residue at the amino-terminal side of the maturation protein was replaced by an alanine residue in order to prevent the binding of fatty acid. Nucleotide sequence of the primer DNA is shown as SEQ ID NO: 10. The segment from position 4 to position 9 in the nucleotide sequence of SEQ ID NO: 10 corresponds to the recognition site for the restriction enzyme PstI, the segment from position 6 to position 17 of the nucleotide sequence corresponds to the amino acid residues, alanine-glycine-serine-serine. The primer DNA was chemically synthesized using a DNA synthesizer. The primer will hereinafter be referred to as "primer 47-1".

A primer DNA for the carboxyl-terminal side was designed so that the primer DNA includes a genetic code for termination of translation and a recognition site for the restriction enzyme BamHI adjacent to and downstream from the code. The nucleotide sequence of the primer DNA is shown as SEQ ID NO: 11. The segment from position 4 to position 9 in the nucleotide sequence shown as SEQ ID NO: 11 corresponds to a recognition site for the restriction enzyme BamHI. The next segment from position 10 to position 12 corresponds to a genetic code for termination of translation. The primer DNA was chemically synthesized by a DNA synthesizer. The primer will hereinafter be referred to as "primer 47-2".

The chromosomal DNA derived from *Treponema pallidum* was used as template DNA. Water(62 µl), template DNA(1 µl), Taq polymerase buffer(10 µl), 1.25mM dNTP (a mixture of equal moles of dATP, dCTP, dGTP and dTTP)(16 µl), 20pmole/µl primer 47-1 in water(5 µl), 20pmole/µl primer 47-2 in water (5 µl) and 1 µl of Taq polymerase (5 units) were mixed. Next, PCR was performed by heating the mixture at 96°C for 0.5 minute and then repeating 25 times the following cycle: heating at 94°C for 1 minute; cooling at 54°C for 1 minute; and holding at 70°C for 1 minute. Portion of the so-reacted solution was digested with restriction enzymes PstI and KpnI and the rest was digested with KpnI and BamHI to give PstI-KpnI fragment (654bp) and KpnI-BamHI fragment (624bp).

Plasmid pNH400 (see J. Bacteriol., Vol.177, pp.745-749(1995)) was digested with restriction enzymes PstI and BamHI. The digest obtained was mixed with the above PstI-KpnI fragment (654bp) and KpnI-BamHI fragment (624bp), and Solution A (20 µl) and Solution B (5 µl) included in Takara DNA ligation Kit (available from Takara Shuzo Co., Ltd.) were added. Plasmid pNH400TP47 of about 5.4kbp length was constructed by subjecting the mixture to a ligation reaction at 16°C for 30 minutes.

### (2) Preparation of transformant comprising plasmid pNH400TP47

The constructed plasmid was introduced into *Bacillus brevis* HPD31 strain using elecrtoporation to give a transformant, *Bacillus brevis* HPD31 strain containing plasmid pNH400TP47. The transformant was deposited as FERM BP-5642 with the National Institute of Bioscience and Human-Technology, Agency of Industrial Science and Technology (Japan).

### (3) Production of Modified Protein

*Bacillus brevis* HPD31 strain containing plasmid pNH400TP47 was applied to a TMN agar plate sterilized by autoclaving for 5 minutes at 121°C (medium at pH7.0 containing 1% peptone, 0.5% meat extract, 0.2% yeast extract, 1% glucose, 0.01% MgSO₄, 0.01% FeSO₄, 0.001% MnSO₄, 0.0001% ZnSO₄, 1.5% agar and 50 µg/ml of neomycin) and grown for 48 hours at 30°C to form colonies. The formed colonies were transferred to 500ml Erlenmeyer flask containing 100ml of TMN solution medium (above TMN agar plate without agar) and cultured with shaking for 2 days at 30°C. The culture solution was centrifuged and the supernatant was collected as a fraction containing the modified protein of the antigenic protein having a molecular weight of about 47 kDa derived from *Treponema pallidum.*

### (4) SDS-polyacrylamide gel electrophoresis of the modified protein-containing fraction

Equivalent volumes of the culture supernatant prepared as described in above (3) and a sample-treatment solution (a mixture solution of 0.0625M Tris-HCl (pH6.8), 2% SDS, 10% glycerol, 5% 2-mercaptoethanol and 0.001% Bromophenol Blue) were mixed well and heated for 5 minutes at 100°C to give a specimen.

The specimen (10 µl) was applied to a polyacrylamide gradient gel with an acrylamide concentration of 10% to 20% (Daiichi Pure Chemicals Co., Ltd.; Product Name: Multigel 10/20,) and subjected to SDS-polyacrylamide gel electrophoresis with a current of 40mA using electrophoresis buffer (25mM Tris buffer containing 250mM glycine and 0.1% SDS, pH8.3). Two lanes were used for sample run. Molecular weight markers also were run.

### (5) Confirmation of the modified protein

After electrophoresis, the gel containing molecule weight markers and one of the sample lanes was excised and stained with Coomassie Brilliant Blue (CBB).

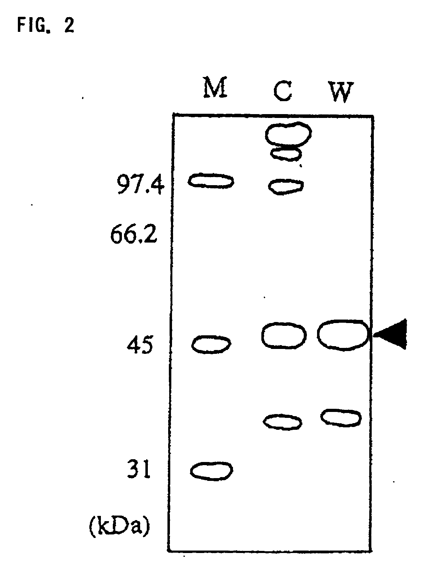

As a result, a blue band was found at the position corresponding to a molecular weight of about 47 kDa as shown in Fig. 2 (Lane C), indicating that the above culture supernatant contained the protein having a molecular weight of about 47 kDa.

### (6) Examination of antigenicity of the modified protein by Western Blotting

On the other hand, the rest of the gel was dipped in a transcription buffer (an aqueous solution containing 0.02M Tris, 0.15M glycine and 20% ethanol) for 15 minutes and equilibrated therewith. Subsequently, the gel was placed on a similarly equilibrated nitrocellulose membrane (Bio-Rad Laboratories, Inc.) and sandwiched between similarly equilibrated filters. The sandwiched gel was then set on a transcription apparatus (Bio-Rad Laboratories, Inc.) and applied with a constant voltage of 100V for one hour to tranfer the protein contained in the gel to the nitrocellulose membrane. After the transfer, the nitrocellulose was dipped in 1% bovine serum albumin (hereinafter referred to as BSA) solution (KPL Corporation; Product Name: 10-fold concentrated solution for BSA dilution/blocking) and left overnight at 4°C.

The resulting nitrocellulose membrane was washed with a washing buffer (0.015M Tris, 0.2M NaCl, 0.05% Tween20) for 10 minutes with exchanging it with the fresh buffer every two minutes. The nitrocellulose membrane was cut into a strip of about 5mm width for each lane and placed in a grooved reaction container. One ml of serum from a syphilis patient which had been diluted 100-fold with a serum dilution buffer (an aqueous solution of 0.015M Tris, 0.2M NaCl and 0.2% BSA) was added into the reaction container and reacted with shaking for 2 hours. After reaction, the nitrocellulose membrane was washed with the washing buffer solution with shaking for 35 minutes while the washing buffer was exchanged after 15 minutes and then every 5 minutes. Then, the nitrocellulose membrane was placed into the reaction container and reacted with 1 ml of peroxidase labeled anti-human IgG antibody (KPL Corp.) which had been diluted 500-fold with serum dilution buffer for 2 hours under shaking. After reaction, the nitrocellulose membrane was washed with the washing buffer for 35 minutes under shaking while the washing buffer was exchanged after 15 minutes and then every five minutes. Then, the nitrocellulose membrane was dipped for 10 minutes in a diaminobenzidine solution (a solution containing 0.05M Tris, 0.9% NaCl (20ml), 31% hydrogen peroxide (32 µl) and diaminobenzidine (solution) (10mg)) prepared when used. The nitrocellulose membrane was then washed well with distilled water and dried.

As a result, a brown band was identified at the position corresponding to a molecular weight of about 47 kDa as shown in Fig. 2 (Lane W), indicating that the serum from the syphilis patient reacted with the modified protein of the antigenic protein having a molecular weigh of about 47 kDa derived from *Treponema pallidum.* It could be assumed from the density of the band that the yield of the modified protein was about 1 g per 1 liter of the culture fluid.

On the other hand, the serum from a healthy person instead of the serum from the syphilis patient was subjected to the same analysis. No band was identified, indicating that the above modified protein did not react with the healthy person's serum.

From the above results, it was demonstrated that the modified protein prepared according to the present invention of the antigenic protein having a molecular weight of about 47 kDa derived from *Treponema pallidum* reacted specifically with the syphilis patient serum.

### (7) Assay of anti-Treponema pallidum antibody by ELISA

The culture supernatant obtained as described in above (3) was added with ammonium sulfate at the concentration of 25% while it was gently stirred. The mixture was stirred overnight at 4°C and then 1 hour at 37°C and centrifuged at 10,000×g for 30 minutes to give a precipitate. Then, the precipitate was washed twice by suspending in 50mM Tris-HCl buffer containing 25% ammonium sulfate (pH8.6) and centrifuging under the above conditions. The resulting precipitate was dissolved in a small amount of a buffer (50mM Tris-HCl buffer containing 1% SDS, pH8.6) to give a protein solution.

An antigen solution containing 0.95 µg/ml of the protein and 0.02% SDS was prepared by diluting the protein solution with 50mM Tris-HCl buffer (pH8.6). The antigen solution (100 µl) was poured into wells of a microtiter plate and left to stand overnight at 4°C.

The antigen solution was removed by aspiration from the wells which were then washed twice with 250 µl of a washing solution (phosphate-buffered saline (PBS) containing 0.05% Tween 20 and 0.1% NaN₃).

Subsequently, 250 µl of a blocking solution (KPL Co. Ltd., 2-fold dilution of 10-fold concentrated bovine serum albumin(BSA) with PBS) was poured into the wells, left at 37°C for one hour, and then removed by aspiration.

Two hundred-fold dilution of a serum from a syphilis patient diluted with the above washing solution (100 µl) was poured into the wells, left at 37°C for 1 hour, and removed by aspiration from the wells. Then, the wells were washed three times with 250 µl of the above washing solution.

Further, 100 µl of a 1000-fold diluted solution (secondary antibody solution) prepared by diluting a goat anti- human IgG monoclonal antibody (KPL Co., Ltd) labeled with alkaline phosphatase with a diluting solution (an aqueous solution containing Tris (0.606g), MgCl₂·H₂O(20.3mg), NaN₃ (0.1g), BSA (5g), Tween 20 (50 µl), goat serum (0.3ml) and glycerin (10ml), total amount of 100ml, adjusted to pH 8.0 with HCl) was poured into the wells, left at 37°C for one hour, and removed by aspiration from the wells. The wells were washed three times with 250 µl of the above washing solution.

Finally, 100 µl of a coloring reagent (prepared by dissolving one substrate tablet (KPL Co., Ltd. containing 10 mg of p-nitrophenyl phosphate) in a dilution prepared by 5-fold diluting 1 ml of a solution in total volume of 270ml containing MgCl₂ · 6H₂O (0.1374g) and diethanolamine (141.9ml) with distilled water) was poured into the wells and left for 10 minutes at room temperature so that the color of the reagent was developed. Then, 3N NaOH (25 µl) was poured into the wells to stop the color development and the absorbance was determined at 405nm using a microplate reader (Tosoh Corporation) to be 0.425.

The result shows that anti-*Treponema pallidum* antibodies in sera from syphilis patient could be assayed by ELISA utilizing, as an antigen, the modified protein of the antigenic protein having a molecular weight of about 47 kDa derived from *Treponema pallidum* obtained according to the present invention.

### Example 2: Preparation of a modified protein of the antigenic protein having a molecular weight of about 17 kDa derived from Treponema pallidum, examination of the antigenicity of the modified protein by Western blotting, and assay of anti-Treponema pallidum antibodies by ELISA

### (1) Preparation of a recombinant vector which expresses a modified protein of the antigenic protein having a molecular weight of about 17 kDa derived from Treponema pallidum

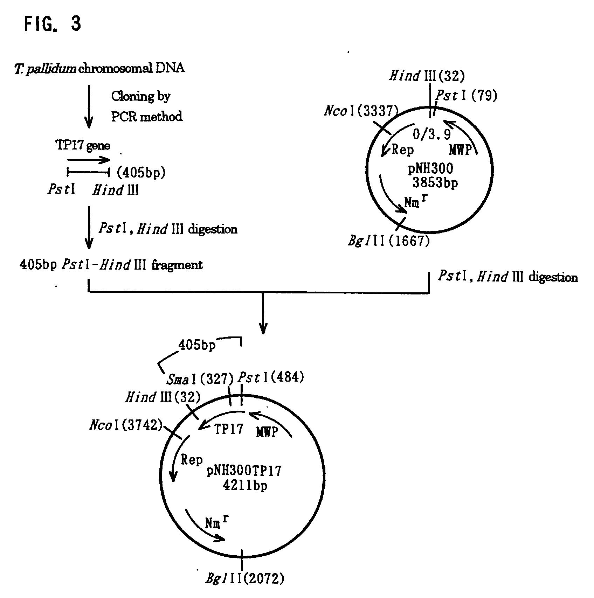

The recombinant vector was constructed according to the procedures shown in Fig. 3.

Akins et al.(*supra*) disclosed a nucleotide sequence of DNA encoding the antigenic protein having a molecular weight of about 17 kDa derived from *Treponema pallidum* (shown as SEQ ID NO: 12).

It is contemplated that the antigenic protein having a molecular weight of about 17 kDa becomes a cell membrane protein in *Treponema* *pallidum* by a mechanism described below. First, a precursor protein comprising a signal sequence (the 1-156 amino acid sequence shown in combination with the nucleotide sequence therefor in SEQ ID NO: 12) is synthesized in a *Treponema pallidum* cell, and a fatty acid present in the cell binds to the cysteine residue at position 22 of the precursor protein via a thioester linkage. The 1-21 amino acid sequence of the precursor protein is removed as the signal sequence by a signal peptidase present in the cell. The bound fatty acid is fixed in the lipid bilayer of the cell membrane, thereby fixing the remaining amino acid sequence starting with amino acid 22 of the precursor protein (i.e., maturation protein) as a cell membrane protein.

Since the fatty acid is considered to prevent the expression/secretion of the antigenic protein, a primer DNA for amino-terminal side was designed so that the cysteine residue at the amino- terminus of the maturation protein was replaced by an alanine residue in order to prevent the binding of the fatty acid. The nucleotide sequence of the primer DNA is shown as SEQ ID NO: 13. In the nucleotide sequence shown as SEQ ID NO: 13, the segment from position 4 to position 9 corresponds to the recognition site for the restriction enzyme PstI, and the segment from position 6 to position 14 corresponds to the amino acid residues, alanine-valine-serine. The primer DNA was chemically synthesized using a DNA synthesizer. The primer DNA will be referred to as "primer 17-1".

On the other hand, a primer DNA for the carboxyl-terminal side was designed so that the primer DNA included a genetic code for termination of translation and a recognition site for the restriction enzyme Hind III adjacent to and downstream from the code. The nucleotide sequence of the primer DNA is shown as SEQ ID NO: 14. In the nucleotide sequence shown as SEQ ID NO: 14, the segment from position 4 to position 9 corresponds to the recognition site for the restriction enzyme Hind III, and the segment from position 8 to position 10 corresponds to the genetic code for termination of translation. The primer DNA was chemically synthesized using a DNA synthesizer. The primer DNA will be referred to as "primer 17-2".

PCR was carried out as in Example 1(1) by using the chromosomal DNA obtained in Example 1(1) as template DNA and using primer 17-1 and primer 17-2 as primer DNAs.

The resulting PCR reaction mixture was digested with restriction enzymes HindIII and PstI to give a HindIII-PstI fragment (405bp) (hereinafter referred to as H-P fragment).

Primer DNA for the amino-terminal side as shown as SEQ ID NO: 15 and primer DNA for the carboxyl-terminal side shown as SEQ ID NO: 16 were designed so that 10 µg of plasmid pNU210 (H. Yamagata, et al., "protein, nucleic acid and enzyme", vol. 37, pp. 258-268 (1992)) can be used as template DNA in order to amplify a portion of the promoter derived from a gene encoding a cell wall protein (MWP) of *Bacillus brevis* present in the plasmid pNU210, a signal sequence of MWP, and a multi-cloning site (Yamagata, H., et al., *J. Bacteriol*., Vol. 169, pp.1245-1289, 1987). Those primer DNAs were synthesized using a DNA synthesizer.

The above plasmid pNU210 (1 µl) as template DNA, water (62 µl), Taq polymerase buffer (10 µl), 1.25mM dNTP (a mixture of equal moles of dATP, dCTP, dGTP and dTTP) (16 µl), 20 pmole/µl of the amino-terminal primer DNA in water (5 µl), 20pmole/µl of the carboxyl-terminal primer DNA in water (5 µl) and Taq polymerase (1 µl, 5 units) were mixed. Next, PCR was performed by heating the mixture at 96°C for 0.5 minute and then repeating 25 times the following cycle: heating at 94°C for 1 minute; cooling at 54°C for 1 minute; and holding at 70°C for 1 minute. The resulting PCR reaction mixture was digested with restriction enzymes SmaI and EcoRI.

On the other hand, plasmid pUB110 (Takara Shuzo, Co., Ltd.) was digested with restriction enzymes EcoRI and PvuII, the resulting digests were mixed with the above digests obtained with restriction enzymes SmaI and EcoRI, and Solution A (20 µl) and Solution B (5 µl) included in Takara DNA Ligation Kit (Takara Shuzo, Co., Ltd.) were added. The mixture was held at 16°C for 30 minutes for ligation reaction to give plasmid pNH300. The plasmid pNH300 was digested with restriction enzymes HindIII and PstI.

Plasmid pNH300TP17 of about 4.2kbp length was constructed by mixing the digests with the above H-P fragment and subjecting the mixture to a ligation reaction using the Ligation Kit in the same manner as described above.

### (2) Preparation of a transformant containing plasmid pNH300TP17

The constructed plasmid was introduced in *Bacillus brevis* HPD31 strain using electroporation to obtain a transformant (*Bacillus brevis* HPD31 strain containing plasmid pNH300TP17). The transformant was deposited as FERM BP-5641 with the National Institute of Bioscience and Human-Technology, Agency of Industrial Science and Technology (Japan).

### (3) Production of the modified protein

A fraction containing the modified protein of the antigenic protein having a molecular weight of about 17 kDa derived from *Treponema pallidum* was obtained by the same procedure as in Example 1 (3) except using *Bacillus brevis* HPD31 strain containing above plasmid pNH300TP17 as the transformant.

### (4) SDS-polyacrylamide gel electrophoresis of the modified protein-containing fraction.

The same procedure as in Example 1 (4) was repeated except using the culture supernatant obtained in the above Example 2(3).

### (5) Confirmation of the modified protein

After electrophoresis, a gel containing molecular weight markers and a sample lane was excised and stained with Coomassie Brilliant Blue (CBB) as in Example 1(5).

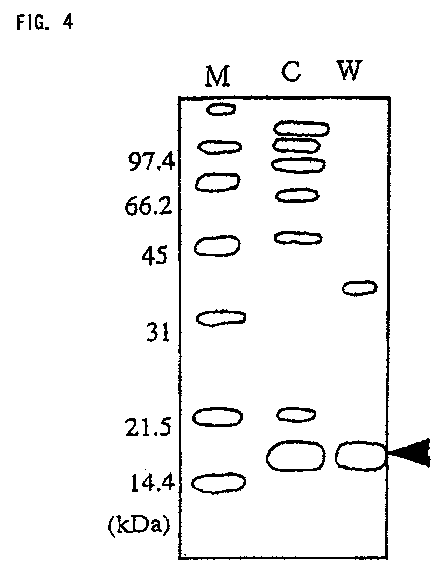

As a result, a blue band was identified at the position corresponding to a molecular weight of about 17 kDa as shown in Fig. 4 (Lane C), indicating that the above described supernatant contained a protein having a molecular weight of about 17 kDa.

### (6) Examination of antigenicity of the modified protein by Western blotting

Western blotting was performed as described in Example 1(6).

As a result, a brown band was identified at the position corresponding to a molecular weight of about 17 kDa as shown in Fig. 4 (Lane W), indicating that the serum from the syphilis patient reacted with the modified protein of the antigenic protein having a molecular weight of 17 kDa derived from *Treponema pallidum*. It could be assumed from the density of the band that the yield of the modified protein was about 1g per 1 liter of the culture fluid.

On the other hand, no band was identified when the same procedure was performed except using a healthy person's serum instead of the serum from the syphilis patient, indicating that the modified protein did not react with the healthy person's serum.

The above results showed that the modified protein prepared according to the present invention of the antigenic protein having a molecular weight of about 17 kDa derived from *Treponema pallidum* reacted specifically with the serum from the syphilis patient.

### (7) Assay of anti-Treponema pallidum antibody using ELISA

The procedure described in the above Example 1(7) was repeated except using the culture supernatant obtained in Example 2(3). An antigen solution, which contained 0.3 µg/ml protein and 0.02% SDS, was prepared using 50 mM Tris-HCl buffer (pH8.6) and a small amount of SDS so that the antigen solution contained the same mole of the protein as that of the protein included in the antigen solution used in Example 1(7).

Then, the absorbance of the solution after its color developed was determined at 405 nm as described in Example 1(7) to be 0.828.

The result shows that anti-*Treponema pallidum* antibody in the serum from the syphilis patient could be assayed by ELISA using the modified protein of the antigenic protein having a molecular weight of about 17 kDa derived form *Treponema pallidum* obtained according to the present invention.

### Example 3: Assay of anti-Treponema pallidum antibody by ELISA using a mixture of the modified protein of the antigenic protein of about 47 kDa from Treponema pallidum and the modified protein of the antigenic protein of about 17 kDa from Treponema pallidum

The culture supernatant obtained in Example 1(3) was added with ammonium sulfate at a concentration of 25% while it was gently stirred. The mixture was stirred overnight at 4°C and then for 1 hour at 37°C, and centrifuged at 10,000×g for 30 minutes to give a precipitate. The resulting precipitate was washed twice by suspending the precipitate in 50 mM Tris-HCl buffer containing 25% ammonium sulfate (pH8.6) and centrifuging it under above conditions. The resulting precipitate was dissolved in a small amount of a buffer (50mM Tris-HCl buffer containing 1% SDS, pH 8.6) to give a protein solution.

On the other hand, the culture supernatant prepared in Example 2(3) was fractionated by cationic column chromatography and a fraction containing the modified protein of the antigenic protein having a molecular weight of about 17 kDa was obtained using the above-described Western blotting.

After the protein solution and the modified protein-containing fraction were mixed, the mole number of the protein contained in the mixture was adjusted so as to be equal to that of the protein contained in the antigen solution of Example 1(7). Then, an antigen solution containing 1.25 µl/ml protein and 0.02% SDS was prepared using 50 mM Tris-HCl buffer (pH8.6) containing 1% SDS. Subsequently, the same procedure as described in Example 1(7) was repeated to determine the absorbance of the solution at 405nm after its color development to be 0.925.

### Example 4: Preparation of a modified protein wherein the antigenic protein having a molecular weight of about 47 kDa derived from Treponema palildum was fused with the antigenic protein having a molecular weight of about 17 kDa derived from Treponema pallidum, examination of antigenicity of the fusion protein by Western blotting, and assay of anti-Treponema pallidum antibody by ELISA

### (1) Preparation of a recombinant plasmid which expresses the modified protein prepared by fusing the antigenic protein having a molecular weight of about 47 kDa with the antigenic protein having a molecular weight of about 17 kDa, the antigenic proteins being both derived from Treponema pallidum.

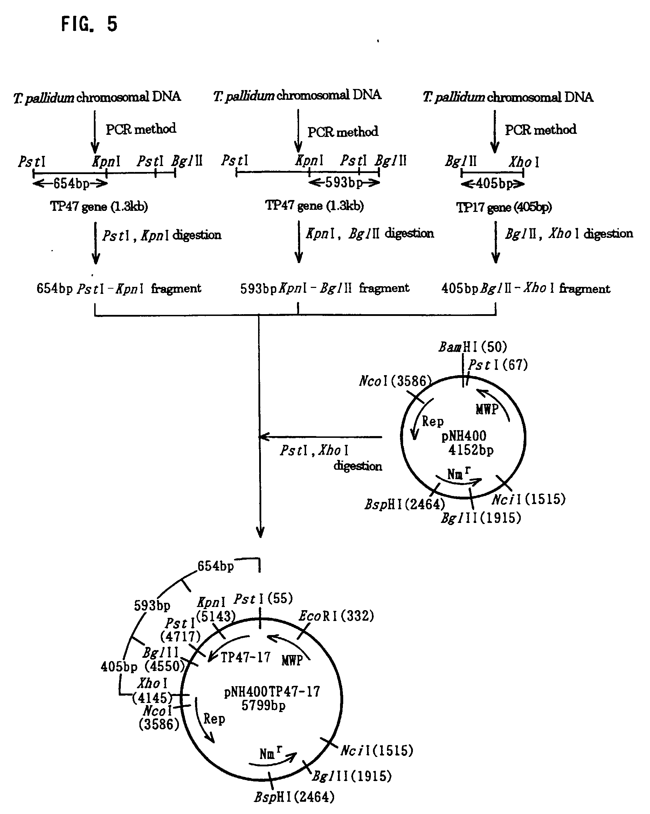

A recombinant plasmid was prepared according to the procedure shown in Fig. 5

As *Treponema pallidum*, *Treponema pallidum* Nichols strain (available from Lee Laboratories, U.S.A.: Product Code: SPROJ-TPURE) was used.

Genomic DNA of *Treponema pallidum* was obtained by the method of M.V. Norgard et al. (*supra*) as follows:

*Treponema pallidum* cells (5×10⁷) were suspended in an aqueous solution containing 1 M sodium chloride, 1 N sodium hydroxide and 2% SDS and boiled for 1 minute. The aqueous solution was then neutralized by adding 0.5 M Tris (pH7.0) in the amount of four times the volume of the solution. Further, phenol was added to the resulting solution to give an aqueous phase to which ethanol was added. Then the mixture was centrifuged to give a precipitate which was dried. Finally, the precipitate was dissolved in sterilized water to form a solution of genomic DNA derived from *Treponema pallidum*. The genomic DNA solution was used as template DNA in PCR method as described below.

Weigel et al. (*supra*) disclosed a nucleotide sequence of DNA which encodes a native antigenic protein from *Treponema pallidum* having a molecular weight of about 47 kDa (shown as SEQ ID NO: 9).

The native antigenic protein having a molecular weight of about 47 kDa is assumed to become a cell membrane protein in *Treponema pallidum* cells by a mechanism described below. First, a precursor protein comprising a signal sequence (the 1-434 amino acid sequence shown in combination with the nucleotide sequence therefor in SEQ ID NO: 9) is synthesized in a *Treponema pallidum* cell. Then, a fatty acid present in the cell binds to the cysteine residue at position 20 of the precursor protein via a thioester linkage. The 1-19 amino acid sequence of the precursor protein is removed as a signal sequence by a signal peptidase present in the cell, the bound fatty acid is fixed in the lipid bilayer of the cell membrane, and the amino acid sequence starting with amino acid 20 in the amino acid sequence of the precursor protein (i.e., maturation protein) is thereby fixed as a cell membrane protein.

Since the fatty acid is assumed to prevent the expression/secretion of the antigenic protein, a primer DNA for the amino-terminal side was designed so that the cysteine residue at the amino-terminal of the maturation protein is replaced by an alanine residue in order to prevent the binding of fatty acid. The nucleotide sequence of the primer DNA is shown as SEQ ID NO: 17. The sequence from position 4 to position 9 in the nucleotide sequence of SEQ ID NO: 17 is the recognition site for restriction enzyme PstI. The primer DNA was chemically synthesized using a DNA synthesizer. The primer will hereinafter be referred to as "primer 47-17-1".

The nucleotide sequence shown as SEQ ID NO: 18 was designed as a primer DNA for the carboxyl-terminal side. The sequence from position 4 to position 9 of the nucleotide sequence shown as SEQ ID NO: 18 is the recognition site for restriction enzyme BgIII. The primer DNA was chemically synthesized by a DNA synthesizer. The primer will hereinafter be referred to as "primer 47-17-2".

Chromosomal DNA from *Treponema pallidum* obtained in the above Example 1(1) was used as template DNA. Water (62 µl), template DNA (1 µl), Taq polymerase buffer (10 µl), 1.25mM dNTP (a mixture containing equal moles of dATP, dCTP, dGTP and dTTP) (16 µl), 20 pmole/µl primer 47-17-1 in water(5 µl), 20pmole/µl primer 47-17-2 in water(5 µl) and Taq polymerase (1 µl, 5 units) were mixed. PCR was performed by heating the mixture at 96°C for 0.5 minute and then repeating 25 timed the following cycle: heating at 94°C for 1 minute; cooling at 54°C for 1 minute; and holding at 70°C for 1 minute. Portion of the resulting reaction solution was digested with restriction enzymes PstI and KpnI, and the rest with restriction enzymes KpnI and BglII to give PstI-KpnI fragment(654bp)and KpnI-BglII fragment(593bp).

On the other hand, D.R. Akins et al., *supra*, disclosed a nucleotide sequence of DNA which encodes a native antigenic protein having a molecular weight of about 17 kDa derived from *Treponema pallidum* (shown as SEQ ID NO: 12).

The nucleotide sequence shown as SEQ ID NO: 19 was designed as a primer DNA for the amino-terminal side. The sequence from position 4 to position 9 of the nucleotide sequence of SEQ ID NO: 19 corresponds to the recognition site for the restriction enzyme BglII. The primer DNA was synthesized using a DNA synthesizer. The primer DNA will be referred to as "primer 47-17-3".

The nucleotide sequence shown as SEQ ID NO: 20 was designed as a primer DNA for the carboxyl-terminal side. The nucleotide sequence from position 4 to position 9 of SEQ ID NO: 20 corresponds to the recognition site for the restriction enzyme XhoI. The primer DNA was synthesized using a DNA synthesizer. The primer DNA will be referred to as "primer 47-17-4".

Then, PCR was performed in the same manner as described above using the template DNA described above and the primers 47-17-3 and 47-17-4.

The resulting PCR reaction mixture was digested with restriction enzymes BglII and XhoI to give a BglII-XhoI fragment (405bp).

Plasmid pNH400 (see *J. Bacteriol*., Vol. 177, pp. 745-749, 1995) was digested with restriction enzymes PstI and XhoI. The resulting digests, the PstI-KpnI fragment (654bp), the KpnI-BglII fragment (593bp) and the BglII-XhoI fragment (405bp) were mixed together. Then, the mixture was added with solution A (20 µl) and solution B(5 µl) included in Takara DNA Ligation Kit (Takara Shuzo Co., Ltd.), and plasmid pNH400TP47-17 having a size of about 5.8kbp was constructed by holding the mixture at 16°C for 30 minutes for ligation reaction.

### (2) Preparation of a transformant comprising plasmid pNH400TP47-17

The constructed plasmid was introduced into *Bacillus brevis* HPD31 strain by the electroporation method to give a transformant (*Bacillus brevis* HPD31 strain containing plasmid pNH400TP47-17). The transformant was deposited as FERM BP-5763 with the National Institute of Bioscience and Human-Technology, Agency of Industrial Science and Technology (Japan).

### (3) Production of the modified protein

TMN agar plate (a medium containing 1% peptone, 0.5% meat extract, 0.2% yeast extract, 1% glucose, 0.01% MgSO₄, 0.01% FeSO₄, 0.001% MnSO₄, 0.0001% ZnSO₄, 1.5% agar, 50 µg/ml neomycin, pH7.0) was sterilized by autoclaving at 12°C for 5 minutes. *Bacillus brevis* HPD31 strain carrying plasmid pNH400TP47-17 was inoculated on the TMN agar plate and grown at 30°C for 48 hours to form colonies. The formed colonies were transferred to a 500ml-Erlenmeyer flask containing a TMN liquid medium (TMN agar plate without agar)(100ml) and grown under shaking at 30°C for 2 days. The culture was centrifuged to recover the supernatant which was a fraction containing the modified protein, i.e., fusion protein of the antigenic protein having a molecular weight of about 47 kDa and the antigenic protein having a molecular weight of about 17 kDa derived from *Treponema pallidum.*

### (4) SDS-polyacrylamide gel electrophoresis of the modified protein-containing fraction

Equal volumes of the culture supernatant obtained in the above Example 4(3) and a sample treatment solution (mixture of 0.0625M Tris-HCl (pH6.8), 2% SDS, 10% glycerol, 5% 2-mercaptoethanol and 0.001% bromophenol blue) were well mixed and heated at 100°C for 5 minutes to give a specimen.

The specimen (10 µl) was applied to a polyacrylamide gel gradient gel containing 10%-20% acrylamide (DAIICHI PURE CHEMICALS Co., Ltd., Product Code: Multigel 10/20), and SDS-polyacrylamide gel electrophoresis was performed at a current of 40mA using a running buffer (25mM Tris buffer containing 250mM glycerine and 0.1% SDS, pH8.3). Two lanes were used for the sample. Molecular weight markers were also run concurrently with the specimen.

### (5) Confirmation of the modified protein

After electrophoresis, a gel containing molecular weight markers and a sample lane was excised and stained with Coomassie Brilliant Blue (CBB).

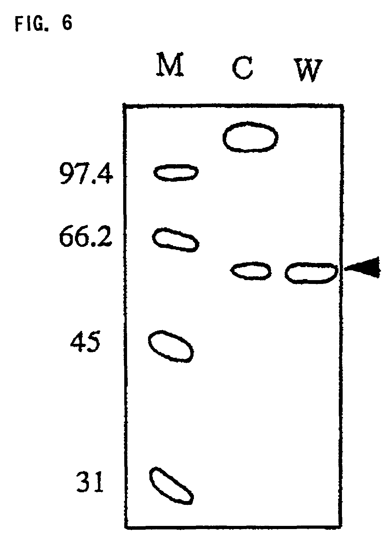

As shown in Fig. 6, the blue band identified at the position corresponding to a molecular weight of about 64 kDa (Lane C) shows that the above culture supernatant contained a protein having a molecular weight of about 64 kDa.

### (6) Examination of antigenicity of the modified protein by Western blotting

Western Blotting was performed as described in Example 1(6).

As a result, the brown band identified at the position corresponding to a molecular weight of about 64 kDa as shown in Fig. 6, (Lane W) showed that the serum from the syphilis patient reacted with the modified protein, i.e., the fused protein of the antigenic protein having a molecular weight of about 47 kDa and the antigenic protein having a molecular weight of about 17 kDa derived from *Treponema pallidum.* The yield of the fusion protein was estimated to be about 0.2g per 1 liter of culture fluid from the density of the band.

On the other hand, no band was identified when the same procedure was performed except using a healthy human serum instead of the syphilis patient's serum. This indicates that the fusion protein did not react with the healthy person's serum.

The above results show that the modified protein prepared according to the present invention by fusing the antigenic protein from *Treponema pallidum* having a molecular weight of about 47 kDa with the antigenic protein from *Treponema pallidum* having a molecular weight of about 17 kDa reacted specifically with the serum from the syphilis patient.

### (7) Assay of anti-Treponema pallidum antibody by ELISA

The culture supernatant obtained in the above Example 4(3) was slowly stirred while ammonium sulfate was added thereto at a concentration of 10%. The mixture was stirred at 4°C overnight and then at 37°C for 1 hour, and centrifuged at 10,000×g for 30 minutes to collect the supernatant. The collected supernatant was added with additional ammonium sulfate at a concentration of 25%. The mixture was stirred at 4°C overnight and then 37°C for 1 hour, and centrifuged at 10,000×g for 30 minutes to give a precipitate. The resulting precipitate was washed twice by suspending it in 50 mM Tris-HCl buffer containing 25% ammonium sulfate (pH 8.6) and centrifuging under the above conditions. The resulting precipitate was dissolved in a small amount of a buffer (50mM Tris-HCl buffer containing 1% SDS, pH8.6) to give a protein solution.

An antigen solution containing 1.25 µg/ml protein and 0.02% SDS was prepared by diluting the protein solution with 50mM Tris-HCl buffer (pH8.6).

The absorbance of the solution at 405nm after color development was determined as in Example 1(7) to be 2.107.

This result showed that anti-*Treponema pallidum* antibodies in the serum from the syphilis patient could be assayed by ELISA using, as an antigen, the modified protein prepared according to the present invention by fusing the antigenic protein from *Treponema pallidum* having a molecular weight of about 47 kDa with the antigenic protein from *Treponema pallidum* having a molecular weight of about 17 kDa.

It is apparent, from the results obtained in Example 3 and Example 4, that the value determined by ELISA using, as an antigen, a mixture of the modified proteins of the antigenic protein having a molecular weight of about 47 kDa and the antigenic protein having a molecular weight of about 17 kDa was 0.925, while the value determined using the modified protein which was the fusion protein of the antigenic protein having a molecular weight of about 47 kDa and the antigenic protein having a molecular weight of about 17 kDa was 2.107, which was much higher than the former.

This showed that the modified protein of the present invention obtained in Example 4 has much higher antigenicity than any antigenic protein derived from *Treponema pallidum* and mixtures thereof.

### Example 5: Preparation of anti-Treponema pallidum antibody using the modified protein as an antigen

Anti-*Treponema pallidum* antibodies can be prepared using as an antigen the modified protein of the present invention, for example, as follows.

### (A) Culture and subculture of myeloma cell line

Myeloma cell line, P3X63Ag8.653 (ATCC CRL-1580) is grown in RPMI1640 medium containing 10% (v/v) fetal calf serum and subcultured at 37°C in the presence of 5% (v/v) CO₂. Two weeks before cell fusion, the cell line is cultured for 1 week in RPMI1640 medium containing 0.13mM 8-azaguanine, 0.5 µg/ml MC-210 (mycoplasma remover, Dainippon Pharmaceutical Co., Ltd.) and 10% (v/v) fetal calf serum, and then in a standard medium for 1 week.

### (B) Immunization of mouse

Suspension of the modified protein according to the present invention containing 270 µg/ml protein (200 µl) is centrifuged at 12000rpm for 10 minutes to give a precipitate. The resulting precipitate is resuspended in 200 µl of PBS. Freund's complete adjuvant is added to the suspension to form an emulsion of which 150 µl is subcutaneously injected in the back of a mouse (day 0). On day 14, day 34 and day 49, the suspension of the above modified protein containing 270 µg/ml protein (100 µl) is intraperitoneally injected in the mouse. Further, the suspension of above modified protein containing 800 µg/ml of the protein (50 µl) and the same suspension (100 µl) are intraperitoneally injected in the mouse on day 69 and day 92, respectively. On day 95, the spleen is removed from the mouse for cell fusion.

### (C) Cell fusion

Splenic cells from the spleen (10⁸) and myeloma cells (10⁷) are transferred to and mixed well in a round bottom glass tube and centrifuged at 1400rpm for 5 minutes. The resulting supernatant is removed and the cells were mixed well. The cells are added to the RPMI1640 medium containing 30% (w/v) polyethylene glycol (0.4ml) preheated at 37°C, and left for 30 seconds. After the mixture is centrifuged at 700rpm for 6 minutes, 10ml of RPMI1640 medium is added to the cells. The glass tube is slowly rotated so that the polyethylene glycol is mixed well with the cells. The mixture is centrifuged at 1400 rpm for 5 minutes to completely remove the supernatant. The precipitates is added with 5 ml of HAT medium and left for 5 minutes. Additional 10-20 ml of HAT medium is added and the mixture is left for 30 minutes. The cells are suspended in the HAT medium so that the medium contains 3.3×10⁵ myeloma cells/ml. The suspension is dispensed to each well of a 96-well plastic culture vessel using a Pasteur pipette in amount of 2 drops/well. The cells are cultured at 36°C under 5% (v/v) carbon dioxide atmosphere, and 1-2 drops of HAT medium are added to each well after 1, 7 and 14 days.

### (D) Screening of antibody-forming cells

The above modified protein is suspended in 0.05M sodium bicarbonate buffer (pH 9.6) containing 0.02% (w/v) NaN₃ at a concentration of 1-10 µg/ml protein. The suspension is dialyzed against 0.05M sodium bicarbonate buffer (pH 9.6) containing 0.02% NaN₃, and diluted so that it contained the protein at a concentration of 1-10 µg/ml. The diluted solution (50 µl) is placed into wells of a 96-well vinyl chloride plate for EIA and left for 4°C overnight to allow the antigen to be absorbed. After the supernatant is removed, 150 µl of PBS containing 0.02% (w/v) Tween 20 was added to the wells. The plate is left for 3 minutes, followed by removal of the PBS and washing (2x). Then, 100 µl of PBS containing 1% (v/v) bovine serum albumin is added to the wells which are then left at 4°C for a period of more than 1 night for blocking. After removal of the PBS containing bovine serum albumin and washing with PBS containing 0.02% (w/v) Tween 20 (2x), 50 µl of the culture supernatant of the fusion cells is added to the wells which are then left for 2 hours at room temperature. The wells are washed three times with PBS containing 0.02% (w/v) Tween 20, followed by addition of 25ng/ml of peroxidase labeled goat anti mouse IgG antibody (50 µl), and left for 2 hours at room temperature. The wells are washed three times with PBS containing 0.02% (w/v) Tween 20, followed by addition of 50 µl of ABTS solution (KPL corporation), and left at room temperature for a period from 15 minutes to 1 hour to develop the color. Then, the absorbance at 405 nm is determined using a photometer for a 96-well EIA plate. Cells in positive wells are collected and transferred to a 24-well plastic culture plate using a Pasteur pipette. The cells are cultured in HAT medium (1-2ml) as described above.

### (E) Cloning by limiting dilution method

Two cell lines of fusion cells are determined for the cell concentration after grown in the 24-well plastic culture plate, and then diluted with HT medium so that the cell number of 20 cells/ml is obtained for each cell line. Thymocyte from 4-6-week-old mouse is suspended in HT medium. The suspension is dispensed to the wells of a 96-well plastic culture plate in an amount of 1-2×10⁵ cells/well to which the fusion cells (20 cells/ml) is then added (50 µl/well). The cells are grown at 36°C under 5% (v/v) carbon dioxide atmosphere, and HT medium (1-2 drops/well) is added to the cultured cells after 1, 7 and 14 days. Culture supernatant (50 µl) is taken from the wells in which cell growth is detected in order to determine if antibodies are produced by the same procedure as in "Screening of antibody-forming cells". Cells which form only a single cell colony per well, produce antibodies that react with the modified protein of the present invention, and grow fast are collected from the wells and subsequently grown in the 24-well plastic culture plate. The cloning process is repeated to give hybridomas which produce anti-*Treponema pallidum* antibodies. Thereafter, anti-*Treponema pallidum* antibodies are produced from the culture supernatant obtained by culturing the hybridomas.

### Effect of the Invention

The modified protein of the present invention can be secreted by microorganisms and has a high production rate with reduced denaturation.

Another modified protein of the present invention has the same advantages as the modified protein described above and a high efficiency of secretion.

Still another modified protein of the present invention has the same advantages as the modified proteins described above and antigenicity as a *Treponema pallidum* antigen.

Still another modified protein of the present invention has the same advantages as the modified proteins described above and antigenicity as a *Treponema pallidum*-specific antigen.

Still another modified protein of the present invention has the same advantages as the modified proteins described above and excellent antigenicity as a *Treponema pallidum*-specific antigen.

Still another modified protein of the present invention has the same advantages as the modified proteins described above and excellent antigenicity as an antigen from any microorganism.

Still another modified protein of the present invention has the same advantages as the modified proteins described above and excellent antigenicity as a *Treponema pallidum*-specific antigen.

Still another modified protein of the present invention has the same advantages as the modified proteins described above and extremely excellent antigenicity as a Treponema pallidum-specific antigen.

The DNA of the present invention is useful for producing a modified protein which can be secreted by microorganisms.

Another DNA of the present invention has the same advantage as the DNA of the present invention described above and is useful for producing a modified protein with excellent antigenicity as a *Treponema pallidum*-specific antigen.

The recombinant vector of the present invention is useful for producing a modified protein which can be secreted by microorganisms.

The transformant of the present invention is useful for producing a modified protein which can be secreted by microorganisms.

Another transformant of the present invention has the same advantage as the transformant of the present invention described above and is useful for producing a modified protein with excellent antigenicity as a *Treponema pallidum*-specific antigen.

The method for producing a modified protein according to the present invention is useful for producing the modified protein which can be secreted by microorganisms.

Another method for producing a modified protein according to the present invention has the same advantages as the method for producing a modified protein of the present invention described above and is useful for conveniently producing a modified protein with excellent antigenicity.

The method for assaying an anti-*Treponema pallidum* antibody according to the present invention is useful for diagnosis of syphilis infection.

The reagent for assaying an anti-*Treponema pallidum* antibody according to the present invention is useful for diagnosis of syphilis infection.

The diagnostic reagent for the syphilis infection of the present invention is useful for diagnosis of syphilis infection.

The process for producing an anti-*Treponema pallidum* antibody according to the present invention is useful for diagnosis of syphilis infection.

Sequence Listing

SEQ ID NO : 1
LENGTH : 415 amino acids
TYPE : amino acid
TOPOLOGY : linear
MOLECULE TYPE : protein

SEQUENCE DESCRIPTION:

Ala Gly Ser Ser His His Glu Thr His Tyr Gly Tyr Ala Thr Leu Ser
1               5                   10                  15
Tyr Ala Asp Tyr Trp Ala Gly Glu Leu Gly Gln Ser Arg Asp Val Leu
            20                  25                  30
Leu Ala Gly Asn Ala Glu Ala Asp Arg Ala Gly Asp Leu Asp Ala Gly
        35                  40                  45
Met Phe Asp Ala Val Ser Arg Ala Thr His Gly His Gly Ala Phe Arg
    50                  55                  60
Gln Gln Phe Gln Tyr Ala Val Glu Val Leu Gly Glu Lys Val Leu Ser
65                  70                  75                  80
Lys Gln Glu Thr Glu Asp Ser Arg Gly Arg Lys Lys Trp Glu Tyr Glu
                85                  90                  95
Thr Asp Pro Ser Val Thr Lys Met Val Arg Ala Ser Ala Ser Phe Gln
            100                 105                 110
Asp Leu Gly Glu Asp Gly Glu Ile Lys Phe Glu Ala Val Glu Gly Ala
        115                 120                 125
Val Ala Leu Ala Asp Arg Ala Ser Ser Phe Met Val Asp Ser Glu Glu
    130                 135                 140

Tyr Lys Ile Thr Asn Val Lys Val His Gly Met Lys Phe Val Pro Val
145                 150                 155                 160
Ala Val Pro His Glu Leu Lys Gly Ile Ala Lys Glu Lys Phe His Phe
                165                 170                 175
Val Glu Asp Ser Arg Val Thr Glu Asn Thr Asn Gly Leu Lys Thr Met
            180                 185                 190
Leu Thr Glu Asp Ser Phe Ser Ala Arg Lys Val Ser Ser Met Glu Ser
        195                 200                 205
Pro His Asp Leu Val Val Asp Thr Val Gly Thr Val Tyr His Ser Arg
    210                 215                 220
Phe Gly Ser Asp Ala Glu Ala Ser Val Met Leu Lys Arg Ala Asp Gly
225                 230                 235                 240
Ser Glu Leu Ser His Arg Glu Phe Ile Asp Tyr Val Met Asn Phe Asn
                245                 250                 255
Thr Val Arg Tyr Asp Tyr Tyr Gly Asp Asp Ala Ser Tyr Thr Asn Leu
            260                 265                 270
Met Ala Ser Tyr Gly Thr Lys His Ser Ala Asp Ser Trp Trp Lys Thr
        275                 280                 285
Gly Arg Val Pro Arg Ile Ser Cys Gly Ile Asn Tyr Gly Phe Asp Arg
    290                 295                 300
Phe Lys Gly Ser Gly Pro Gly Tyr Tyr Arg Leu Thr Leu Ile Ala Asn
305                 310                 315                 320
Gly Tyr Arg Asp Val Val Ala Asp Val Arg Phe Leu Pro Lys Tyr Glu
                325                 330                 335
Gly Asn Ile Asp Ile Gly Leu Lys Gly Lys Val Leu Thr Ile Gly Gly
            340                 345                 350
Ala Asp Ala Glu Thr Leu Met Asp Ala Ala Val Asp Val Phe Ala Asp
        355                 360                 365
Gly Gln Pro Lys Leu Val Ser Asp Gln Ala Val Ser Leu Gly Gln Asn

    370                 375                 380
Val Leu Ser Ala Asp Phe Thr Pro Gly Thr Glu Tyr Thr Val Glu Val
385                 390                 395                 400
Arg Phe Lys Glu Phe Gly Ser Val Arg Ala Lys Val Val Ala Gln
                405                 410                 415

SEQ ID NO : 2
LENGTH : 135 amino acids
TYPE : amino acid
TOPOLOGY : linear
MOLECULE TYPE : protein

SEQUENCE DESCRIPTION:

Ala Val Ser Cys Thr Thr Val Cys Pro His Ala Gly Lys Ala Lys Ala
1               5                   10                  15
Glu Lys Val Glu Cys Ala Leu Lys Gly Gly Ile Phe Arg Gly Thr Leu
            20                  25                  30
Pro Ala Ala Asp Cys Pro Gly Ile Asp Thr Thr Val Thr Phe Asn Ala
        35                  40                  45
Asp Gly Thr Ala Gln Lys Val Glu Leu Ala Leu Glu Lys Lys Ser Ala
    50                  55                  60
Pro Ser Pro Leu Thr Tyr Arg Gly Thr Trp Met Val Arg Glu Asp Gly
65                  70                  75                  80
Ile Val Glu Leu Ser Leu Val Ser Ser Glu Gln Ser Lys Ala Pro His
                85                  90                  95
Glu Lys Glu Leu Tyr Glu Leu Ile Asp Ser Asn Ser Val Arg Tyr Met
            100                 105                 110

Gly Ala Pro Gly Ala Gly Lys Pro Ser Lys Glu Met Ala Pro Phe Tyr
        115                 120                 125
Val Leu Lys Lys Thr Lys Lys
    130                 135

SEQ ID NO : 3
LENGTH : 124 amino acids
TYPE : amino acid
TOPOLOGY : linear
MOLECULE TYPE : protein

SEQUENCE DESCRIPTION:

Ala Ser Phe Ser Ser Ile Pro Asn Gly Thr Tyr Arg Ala Thr Tyr Gln
1               5                   10                  15
Asp Phe Asp Glu Asn Gly Trp Lys Asp Phe Leu Glu Val Thr Phe Asp
            20                  25                  30
Gly Gly Lys Met Val Gln Val Val Tyr Asp Tyr Gln His Lys Glu Gly
        35                  40                  45
Arg Phe Lys Ser Gln Asp Ala Asp Tyr His Arg Val Met Tyr Ala Ser
    50                  55                  60
Ser Gly Ile Gly Pro Glu Lys Ala Phe Arg Glu Leu Ala Asp Ala Leu
65                  70                  75                  80
Leu Glu Lys Gly Asn Pro Glu Met Val Asp Val Val Thr Gly Ala Thr
                85                  90                  95
Val Ser Ser Gln Ser Phe Arg Arg Leu Gly Arg Ala Leu Leu Gln Ser
            100                 105                 110
Ala Arg Arg Gly Glu Lys Glu Ala Ile Ile Ser Arg

115 120 124

SEQ ID NO : 4
LENGTH : 551 amino acids
TYPE : amino acid
TOPOLOGY : linear
MOLECULE TYPE : protein

SEQUENCE DESCRIPTION:

Ala Gly Ser Ser His His Glu Thr His Tyr Gly Tyr Ala Thr Leu Ser
1               5                   10                  15
Tyr Ala Asp Tyr Trp Ala Gly Glu Leu Gly Gln Ser Arg Asp Val Leu
            20                  25                  30
Leu Ala Gly Asn Ala Glu Ala Asp Arg Ala Gly Asp Leu Asp Ala Gly
        35                  40                  45
Met Phe Asp Ala Val Ser Arg Ala Thr His Gly His Gly Ala Phe Arg
    50                  55                  60
Gln Gln Phe Gln Tyr Ala Val Glu Val Leu Gly Glu Lys Val Leu Ser
65                  70                  75                  80
Lys Gln Glu Thr Glu Asp Ser Arg Gly Arg Lys Lys Trp Glu Tyr Glu
                85                  90                  95
Thr Asp Pro Ser Val Thr Lys Met Val Arg Ala Ser Ala Ser Phe Gln
            100                 105                 110
Asp Leu Gly Glu Asp Gly Glu Ile Lys Phe Glu Ala Val Glu Gly Ala
        115                 120                 125
Val Ala Leu Ala Asp Arg Ala Ser Ser Phe Met Val Asp Ser Glu Glu
    130                 135                 140

Tyr Lys Ile Thr Asn Val Lys Val His Gly Met Lys Phe Val Pro Val
145                 150                 155                 160
Ala Val Pro His Glu Leu Lys Gly Ile Ala Lys Glu Lys Phe His Phe
                165                 170                 175
Val Glu Asp Ser Arg Val Thr Glu Asn Thr Asn Gly Leu Lys Thr Met
            180                 185                 190
Leu Thr Glu Asp Ser Phe Ser Ala Arg Lys Val Ser Ser Met Glu Ser
        195                 200                 205
Pro His Asp Leu Val Val Asp Thr Val Gly Thr Val Tyr His Ser Arg
    210                 215                 220
Phe Gly Ser Asp Ala Glu Ala Ser Val Met Leu Lys Arg Ala Asp Gly
225                 230                 235                 240
Ser Glu Leu Ser His Arg Glu Phe Ile Asp Tyr Val Met Asn Phe Asn
                245                 250                 255
Thr Val Arg Tyr Asp Tyr Tyr Gly Asp Asp Ala Ser Tyr Thr Asn Leu
            260                 265                 270
Met Ala Ser Tyr Gly Thr Lys His Ser Ala Asp Ser Trp Trp Lys Thr
        275                 280                 285
Gly Arg Val Pro Arg Ile Ser Cys Gly Ile Asn Tyr Gly Phe Asp Arg
    290                 295                 300
Phe Lys Gly Ser Gly Pro Gly Tyr Tyr Arg Leu Thr Leu Ile Ala Asn
305                 310                 315                 320
Gly Tyr Arg Asp Val Val Ala Asp Val Arg Phe Leu Pro Lys Tyr Glu
                325                 330                 335
Gly Asn Ile Asp Ile Gly Leu Lys Gly Lys Val Leu Thr Ile Gly Gly
            340                 345                 350
Ala Asp Ala Glu Thr Leu Met Asp Ala Ala Val Asp Val Phe Ala Asp
        355                 360                 365
Gly Gln Pro Lys Leu Val Ser Asp Gln Ala Val Ser Leu Gly Gln Asn

    370                 375                 380
Val Leu Ser Ala Asp Phe Thr Pro Gly Thr Glu Tyr Thr Val Glu Val
385                 390                 395                 400
Arg Phe Lys Glu Phe Gly Ser Val Arg Ala Lys Val Val Ala Gln Ile
                405                 410                 415
Cys Val Ser Cys Thr Thr Val Cys Pro His Ala Gly Lys Ala Lys Ala
            420                 425                 430
Glu Lys Val Glu Cys Ala Leu Lys Gly Gly Ile Phe Arg Gly Thr Leu
        435                 440                 445
Pro Ala Ala Asp Cys Pro Gly Ile Asp Thr Thr Val Ser Ser Thr Arg
    450                 455                 460
Met Ala Leu Arg Lys Arg Tyr Glu Leu Ala Leu Glu Lys Lys Ser Ala
465                 470                 475                 480
Pro Ser Pro Leu Thr Tyr Arg Gly Thr Trp Met Val Arg Glu Asp Gly
                485                 490                 495
Ile Val Glu Leu Ser Leu Val Ser Ser Glu Gln Ser Lys Ala Pro His
            500                 505                 510
Glu Lys Glu Leu Tyr Glu Leu Ile Asp Ser Asn Ser Val Arg Tyr Met
        515                 520                 525
Gly Ala Pro Gly Ala Gly Lys Pro Ser Lys Glu Met Ala Pro Phe Tyr
    530                 535                 540
Val Leu Lys Lys Thr Lys Lys
545 550 551

SEQ ID NO : 5
LENGTH : 1286 base pairs
TYPE : nucleic acid
STRANDEDNESS : double

TOPOLOGY : linear

MOLECULE TYPE : other nucleic acid (synthetic DNA)

SEQUENCE DESCRIPTION:

CTGCAGGCTC GTCTCATCAT GAGACGCACT ATGGCTATGC GACGCTAAGC TATGCGGACT 60

ACTGGGCCGG GGAGTTGGGG CAGAGTAGGG ACGTGCTTTT GGCGGGTAAT GCCGAGGCGG 120

ACCGCGCGGG GGATCTCGAC GCAGGCATGT TCGATGCAGT TTCTCGCGCA ACCCACGGGC 180

ATGGCGCGTT CCGTCAGCAA TTTCAGTACG CGGTTGAGGT ATTGGGCGAA AAGGTTCTCT 240

CGAAGCAGGA GACCGAAGAC AGCAGGGGAA GAAAAAAGTG GGAGTACGAG ACTGACCCAA 300

GCGTTACTAA GATGGTGCGT GCCTCTGCGT CATTTCAGGA TTTGGGAGAG GACGGGGAGA 360

TTAAGTTTGA AGCAGTCGAG GGTGCAGTAG CGTTGGCGGA TCGCGCGAGT TCCTTCATGG 420

TTGACAGCGA GGAATACAAG ATTACGAACG TAAAGGTTCA CGGTATGAAG TTTGTCCCAG 480

TTGCGGTTCC TCATGAATTA AAAGGGATTG CAAAGGAGAA GTTTCACTTC GTGGAAGACT 540

CCCGCGTTAC GGAGAATACC AACGGCCTTA AGACAATGCT CACTGAGGAT AGTTTTTCTG 600

CACGTAAGGT AAGCAGCATG GAGAGCCCGC ACGACCTTGT GGTAGACACG GTGGGTACCG 660

TCTACCACAG CCGTTTTGGT TCGGACGCAG AGGCTTCTGT GATGCTGAAA AGGGCTGATG 720

GCTCTGAGCT GTCGCACCGT GAGTTCATCG ACTATGTGAT GAACTTCAAC ACGGTCCGCT 780

ACGACTACTA CGGTGATGAC GCGAGCTACA CCAATCTGAT GGCGAGTTAT GGCACCAAGC 840

ACTCTGCTGA CTCCTGGTGG AAGACAGGAA GAGTGCCCCG CATTTCGTGT GGTATCAACT 900

ATGGGTTCGA TCGGTTTAAA GGTTCAGGGC CGGGATACTA CAGGCTGACT TTGATTGCGA 960

ACGGGTATAG GGACGTAGTT GCTGATGTGC GCTTCCTTCC CAAGTACGAG GGGAACATCG 1020

ATATTGGGTT GAAGGGGAAG GTGCTGACCA TAGGGGGCGC GGACGCGGAG ACTCTGATGG 1080

ATGCTGCAGT TGACGTGTTT GCCGATGGAC AGCCTAAGCT TGTCAGCGAT CAAGCGGTGA 1140

GCTTGGGGCA GAATGTCCTC TCTGCGGATT TCACTCCCGG CACTGAGTAC ACGGTTGAGG 1200

TTAGGTTCAA GGAATTCGGT TCTGTGCGTG CGAAGGTAGT GGCCCAGTAG AAGAGGGGTG 1260

TCCTATCCCG TGTGTCTTAA GGATCC 1286

SEQ ID NO : 6
LENGTH : 416 base pairs
TYPE : nucleic acid
STRANDEDNESS : double
TOPOLOGY : linear
MOLECULE TYPE : other nucleic acid (synthetic DNA)

SEQUENCE DESCRIPTION:

CTGCAGTCTC GTGCACAACC GTGTGTCCGC ACGCCGGGAA GGCCAAAGCG GAAAAGGTAG 60
AGTGCGCGTT GAAGGGAGGT ATCTTTCGGG GTACGCTACC TGCGGCCGAT TGCCCGGGAA 120
TCGATACGAC TGTGACGTTC AACGCGGATG GCACTGCGCA AAAGGTAGAG CTTGCCCTTG 180
AGAAGAAGTC GGCACCTTCT CCTCTTACCT ATCGCGGTAC GTGGATGGTA CGTGAAGACG 240
GAATTGTCGA ACTCTCGCTT GTGTCCTCGG AGCAATCGAA GGCACCGCAC GAGAAAGAGC 300
TGTACGAGCT GATAGACAGT AACTCCGTTC GCTACATGGG CGCTCCCGGC GCAGGAAAGC 360
CTTCAAAGGA GATGGCGCCG TTTTACGTGC TCAAAAAAAC AAAGAAATAG AAGCTT 416

SEQ ID NO : 7
LENGTH : 372 base pairs
TYPE : nucleic acid
STRANDEDNESS : double
TOPOLOGY : linear
MOLECULE TYPE : other nucleic acid (synthetic DNA)

SEQUENCE DESCRIPTION:

GCATCATTTA GTTCTATCCC GAATGGCACG TACCGGGCGA CGTATCAGGA TTTTGATGAG 60
AATGGTTGGA AGGACTTTCT CGAGGTTACT TTTGATGGTG GCAAGATGGT GCAGGTGGTT 120

TACGATTATC AGCATAAAGA AGGGCGGTTT AAGTCCCAGG ACGCTGACTA CCATCGGGTC 180
ATGTATGCAT CCTCGGGCAT AGGTCCTGAA AAGGCCTTCA GAGAGCTCGC CGATGCTTTG 240
CTTGAAAAGG GTAATCCCGA GATGGTGGAT GTGGTCACCG GTGCAACTGT TTCTTCCCAG 300
AGTTTCAGGA GGTTGGGTCG TGCGCTTCTG CAGAGTGCGC GGCGCGGCGA GAAGGAAGCC 360
ATTATTAGCA GG 372

SEQ ID NO : 8
LENGTH : 1664 base pairs
TYPE : nucleic acid
STRANDEDNESS : double
MOLECULE TYPE : other nucleic acid (synthetic DNA)

SEQUENCE DESCRIPTION:

CTGCAGGCTC GTCTCATCAT GAGACGCACT ATGGCTATGC GACGCTAAGC TATGCGGACT 60
ACTGGGCCGG GGAGTTGGGG CAGAGTAGGG ACGTGCTTTT GGCGGGTAAT GCCGAGGCGG 120
ACCGCGCGGG GGATCTCGAC GCAGGCATGT TCGATGCAGT TTCTCGCGCA ACCCACGGGC 180
ATGGCGCGTT CCGTCAGCAA TTTCAGTACG CGGTTGAGGT ATTGGGCGAA AAGGTTCTCT 240
CGAAGCAGGA GACCGAAGAC AGCAGGGGAA GAAAAAAGTG GGAGTACGAG ACTGACCCAA 300
GCGTTACTAA GATGGTGCGT GCCTCTGCGT CATTTCAGGA TTTGGGAGAG GACGGGGAGA 360
TTAAGTTTGA AGCAGTCGAG GGTGCAGTAG CGTTGGCGGA TCGCGCGAGT TCCTTCATGG 420
TTGACAGCGA GGAATACAAG ATTACGAACG TAAAGGTTCA CGGTATGAAG TTTGTCCCAG 480
TTGCGGTTCC TCATGAATTA AAAGGGATTG CAAAGGAGAA GTTTCACTTC GTGGAAGACT 540
CCCGCGTTAC GGAGAATACC AACGGCCTTA AGACAATGCT CACTGAGGAT AGTTTTTCTG 600
CACGTAAGGT AAGCAGCATG GAGAGCCCGC ACGACCTTGT GGTAGACACG GTGGGTACCG 660
TCTACCACAG CCGTTTTGGT TCGGACGCAG AGGCTTCTGT GATGCTGAAA AGGGCTGATG 720
GCTCTGAGCT GTCGCACCGT GAGTTCATCG ACTATGTGAT GAACTTCAAC ACGGTCCGCT 780
ACGACTACTA CGGTGATGAC GCGAGCTACA CCAATCTGAT GGCGAGTTAT GGCACCAAGC 840

ACTCTGCTGA CTCCTGGTGG AAGACAGGAA GAGTGCCCCG CATTTCGTGT GGTATCAACT 900
ATGGGTTCGA TCGGTTTAAA GGTTCAGGGC CGGGATACTA CAGGCTGACT TTGATTGCGA 960
ACGGGTATAG GGACGTAGTT GCTGATGTGC GCTTCCTTCC CAAGTACGAG GGGAACATCG 1020
ATATTGGGTT GAAGGGGAAG GTGCTGACCA TAGGGGGCGC GGACGCGGAG ACTCTGATGG 1080
ATGCTGCAGT TGACGTGTTT GCCGATGGAC AGCCTAAGCT TGTCAGCGAT CAAGCGGTGA 1140
GCTTGGGGCA GAATGTCCTC TCTGCGGATT TCACTCCCGG CACTGAGTAC ACGGTTGAGG 1200
TTAGGTTCAA GGAATTCGGT TCTGTGCGTG CGAAGGTAGT GGCCCAGATC TGTGTCTCGT 1260
GCACAACCGT GTGTCCGCAC GCCGGGAAGG CCAAAGCGGA AAAGGTAGAG TGCGCGTTGA 1320
AGGGAGGTAT CTTTCGGGGT ACGCTACCTG CGGCCGATTG CCCGGGAATC GATACGACTG 1380
TGAGTTCAAC GCGGATGGCA CTGCGCAAAA GGTACGAGCT TGCCCTTGAG AAGAAGTCGG 1440
CACCTTCTCC TCTTACCTAT CGCGGTACGT GGATGGTACG TGAAGACGGA ATTGTCGAAC 1500
TCTCGCTTGT GTCCTCGGAG CAATCGAAGG CACCGCACGA GAAAGAGCTG TACGAGCTGA 1560
TAGACAGTAA CTCCGTTCGC TACATGGGCG CTCCCGGCGC AGGAAAGCCT TCAAAGGAGA 1620
TGGCGCCGTT TTACGTGCTC AAAAAAACAA AGAAATAGCT CGAG 1664

SEQ ID NO : 9
LENGTH : 1332 base pairs
TYPE : nucleic acid
STRANDEDNESS : double
TOPOLOGY : linear
MOLECULE TYPE : Genomic DNA

SEQUENCE DESCRIPTION:

GTG AAA GTG AAA TAC GCA CTA CTT TCT GCC GGA GCG CTG CAG TTG TTG 48
Val Lys Val Lys Tyr Ala Leu Leu Ser Ala Gly Ala Leu Gln Leu Leu
1               5                   10                  15
GTT GTA GGC TGT GGC TCG TCT CAT CAT GAG ACG CAC TAT GGC TAT GCG 96

Val Val Gly Cys Gly Ser Ser His His Glu Thr His Tyr Gly Tyr Ala
            20                  25                  30
ACG CTA AGC TAT GCG GAC TAC TGG GCC GGG GAG TTG GGG CAG AGT AGG 144
Thr Leu Ser Tyr Ala Asp Tyr Trp Ala Gly Glu Leu Gly Gln Ser Arg
        35                  40                  45
GAC GTG CTT TTG GCG GGT AAT GCC GAG GCG GAC CGC GCG GGG GAT CTC 192
Asp Val Leu Leu Ala Gly Asn Ala Glu Ala Asp Arg Ala Gly Asp Leu
    50                  55                  60
GAC GCA GGC ATG TTC GAT GCA GTT TCT CGC GCA ACC CAC GGG CAT GGC 240
Asp Ala Gly Met Phe Asp Ala Val Ser Arg Ala Thr His Gly His Gly
65                  70                  75                  80
GCG TTC CGT CAG CAA TTT CAG TAC GCG GTT GAG GTA TTG GGC GAA AAG 288
Ala Phe Arg Gln Gln Phe Gln Tyr Ala Val Glu Val Leu Gly Glu Lys
                85                  90                  95
GTT CTC TCG AAG CAG GAG ACC GAA GAC AGC AGG GGA AGA AAA AAG TGG 336
Val Leu Ser Lys Gln Glu Thr Glu Asp Ser Arg Gly Arg Lys Lys Trp
            100                 105                 110
GAG TAC GAG ACT GAC CCA AGC GTT ACT AAG ATG GTG CGT GCC TCT GCG 384
Glu Tyr Glu Thr Asp Pro Ser Val Thr Lys Met Val Arg Ala Ser Ala
        115                 120                 125
TCA TTT CAG GAT TTG GGA GAG GAC GGG GAG ATT AAG TTT GAA GCA GTC 432
Ser Phe Gln Asp Leu Gly Glu Asp Gly Glu Ile Lys Phe Glu Ala Val
    130                 135                 140
GAG GGT GCA GTA GCG TTG GCG GAT CGC GCG AGT TCC TTC ATG GTT GAC 480
Glu Gly Ala Val Ala Leu Ala Asp Arg Ala Ser Ser Phe Met Val Asp
145                 150                 155                 160
AGC GAG GAA TAC AAG ATT ACG AAC GTA AAG GTT CAC GGT ATG AAG TTT 528
Ser Glu Glu Tyr Lys Ile Thr Asn Val Lys Val His Gly Met Lys Phe
                165                 170                 175

GTC CCA GTT GCG GTT CCT CAT GAA TTA AAA GGG ATT GCA AAG GAG AAG 576
Val Pro Val Ala Val Pro His Glu Leu Lys Gly Ile Ala Lys Glu Lys
            180                 185                 190
TTT CAC TTC GTG GAA GAC TCC CGC GTT ACG GAG AAT ACC AAC GGC CTT 624
Phe His Phe Val Glu Asp Ser Arg Val Thr Glu Asn Thr Asn Gly Leu
        195                 200                 205
AAG ACA ATG CTC ACT GAG GAT AGT TTT TCT GCA CGT AAG GTA AGC AGC 672
Lys Thr Met Leu Thr Glu Asp Ser Phe Ser Ala Arg Lys Val Ser Ser
    210                 215                 220
ATG GAG AGC CCG CAC GAC CTT GTG GTA GAC ACG GTG GGT ACC GTC TAC 720
Met Glu Ser Pro His Asp Leu Val Val Asp Thr Val Gly Thr Val Tyr
225                 230                 235                 240
CAC AGC CGT TTT GGT TCG GAC GCA GAG GCT TCT GTG ATG CTG AAA AGG 768
His Ser Arg Phe Gly Ser Asp Ala Glu Ala Ser Val Met Leu Lys Arg
                245                 250                 255
GCT GAT GGC TCT GAG CTG TCG CAC CGT GAG TTC ATC GAC TAT GTG ATG 816
Ala Asp Gly Ser Glu Leu Ser His Arg Glu Phe Ile Asp Tyr Val Met
            260                 265                 270
AAC TTC AAC ACG GTC CGC TAC GAC TAC TAC GGT GAT GAC GCG AGC TAC 864
Asn Phe Asn Thr Val Arg Tyr Asp Tyr Tyr Gly Asp Asp Ala Ser Tyr
        275                 280                 285
ACC AAT CTG ATG GCG AGT TAT GGC ACC AAG CAC TCT GCT GAC TCC TGG 912
Thr Asn Leu Met Ala Ser Tyr Gly Thr Lys His Ser Ala Asp Ser Trp
    290                 295                 300
TGG AAG ACA GGA AGA GTG CCC CGC ATT TCG TGT GGT ATC AAC TAT GGG 960
Trp Lys Thr Gly Arg Val Pro Arg Ile Ser Cys Gly Ile Asn Tyr Gly
305                 310                 315                 320
TTC GAT CGG TTT AAA GGT TCA GGG CCG GGA TAC TAC AGG CTG ACT TTG 1008
Phe Asp Arg Phe Lys Gly Ser Gly Pro Gly Tyr Tyr Arg Leu Thr Leu

                325                 330                 335
ATT GCG AAC GGG TAT AGG GAC GTA GTT GCT GAT GTG CGC TTC CTT CCC 1056
Ile Ala Asn Gly Tyr Arg Asp Val Val Ala Asp Val Arg Phe Leu Pro
            340                 345                 350
AAG TAC GAG GGG AAC ATC GAT ATT GGG TTG AAG GGG AAG GTG CTG ACC 1104
Lys Tyr Glu Gly Asn Ile Asp Ile Gly Leu Lys Gly Lys Val Leu Thr
        355                 360                 365
ATA GGG GGC GCG GAC GCG GAG ACT CTG ATG GAT GCT GCA GTT GAC GTG 1152
Ile Gly Gly Ala Asp Ala Glu Thr Leu Met Asp Ala Ala Val Asp Val
    370                 375                 380
TTT GCC GAT GGA CAG CCT AAG CTT GTC AGC GAT CAA GCG GTG AGC TTG 1200
Phe Ala Asp Gly Gln Pro Lys Leu Val Ser Asp Gln Ala Val Ser Leu
385                 390                 395                 400
GGG CAG AAT GTC CTC TCT GCG GAT TTC ACT CCC GGC ACT GAG TAC ACG 1248
Gly Gln Asn Val Leu Ser Ala Asp Phe Thr Pro Gly Thr Glu Tyr Thr
                405                 410                 415
GTT GAG GTT AGG TTC AAG GAA TTC GGT TCT GTG CGT GCG AAG GTA GTG 1296
Val Glu Val Arg Phe Lys Glu Phe Gly Ser Val Arg Ala Lys Val Val
            420                 425                 430
GCC CAG TAG AAG AGG GGT GTC CTA TCC CGT GTG TCT 1332
Ala Gln End Lys Arg Gly Val Leu Ser Arg Val Ser
435 440 444

SEQ ID NO : 10
LENGTH : 36 base pairs
TYPE : nucleic acid
STRANDEDNESS : single
TOPOLOGY : linear

MOLECULE TYPE : other nucleic acid (synthetic DNA)

SEQUENCE DESCRIPTION:

TAGCTGCAGG CTCGTCTCAT CATGAGACGC ACTATG 36

SEQ ID NO : 11
LENGTH : 36 base pairs
TYPE : nucleic acid
STRANDEDNESS : single
TOPOLOGY : linear
MOLECULE TYPE : other nucleic acid (synthetic DNA)

SEQUENCE DESCRIPTION:

AAAGGATCCT TAAGACACAC GGGATAGGAC ACCCCT 36

SEQ ID NO : 12
LENGTH : 468 base pairs
TYPE : nucleic acid
STRANDEDNESS : double
TOPOLOGY : linear
MOLECULE TYPE : Genomic DNA

SEQUENCE DESCRIPTION:

ATG AAA GGA TCT GTC CGC GCG CTG TGC GCG TTC CTT GGT GTT GGA GCG 48

Met Lys Gly Ser Val Arg Ala Leu Cys Ala Phe Leu Gly Val Gly Ala
1               5                   10                  15
CTC GGT AGC GCT TTG TGT GTC TCG TGC ACA ACC GTG TGT CCG CAC GCC     96
Leu Gly Ser Ala Leu Cys Val Ser Cys Thr Thr Val Cys Pro His Ala
            20                  25                  30
GGG AAG GCC AAA GCG GAA AAG GTA GAG TGC GCG TTG AAG GGA GGT ATC     144
Gly Lys Ala Lys Ala Glu Lys Val Glu Cys Ala Leu Lys Gly Gly Ile
        35                  40                  45
TTT CGG GGT ACG CTA CCT GCG GCC GAT TGC CCG GGA ATC GAT ACG ACT     192
Phe Arg Gly Thr Leu Pro Ala Ala Asp Cys Pro Gly Ile Asp Thr Thr
    50                  55                  60
GTG ACG TTC AAC GCG GAT GGC ACT GCG CAA AAG GTA GAG CTT GCC CTT     240
Val Thr Phe Asn Ala Asp Gly Thr Ala Gln Lys Val Glu Leu Ala Leu
65                  70                  75                  80
GAG AAG AAG TCG GCA CCT TCT CCT CTT ACC TAT CGC GGT ACG TGG ATG     288
Glu Lys Lys Ser Ala Pro Ser Pro Leu Thr Tyr Arg Gly Thr Trp Met
                85                  90                  95
GTA CGT GAA GAC GGA ATT GTC GAA CTC TCG CTT GTG TCC TCG GAG CAA     336
Val Arg Glu Asp Gly Ile Val Glu Leu Ser Leu Val Ser Ser Glu Gln
            100                 105                 110
TCG AAG GCA CCG CAC GAG AAA GAG CTG TAC GAG CTG ATA GAC AGT AAC     384
Ser Lys Ala Pro His Glu Lys Glu Leu Tyr Glu Leu Ile Asp Ser Asn
        115                 120                 125
TCC GTT CGC TAC ATG GGC GCT CCC GGC GCA GGA AAG CCT TCA AAG GAG     432
Ser Val Arg Tyr Met Gly Ala Pro Gly Ala Gly Lys Pro Ser Lys Glu
    130                 135                 140
ATG GCG CCG TTT TAC GTG CTC AAA AAA ACA AAG AAA                     468
Met Ala Pro Phe Tyr Val Leu Lys Lys Thr Lys Lys
145                 150                 155 156

SEQ ID NO : 13
LENGTH : 32 base pairs
TYPE : nucleic acid
STRANDEDNESS : single
TOPOLOGY : linear
MOLECULE TYPE : other nucleic acid (synthetic DNA)

SEQUENCE DESCRIPTION:

AAACTGCAGT CTCGTGCACA ACCGTGTGTC CG 32

SEQ ID NO : 14
LENGTH : 32 base pairs
TYPE : nucleic acid
STRANDEDNESS : single
TOPOLOGY : linear
MOLECULE TYPE : other nucleic acid (synthetic DNA)

SEQUENCE DESCRIPTION:

AAAAAGCTTA TTTCTTTGTT TTTTTGAGCA CG 32

SEQ ID NO : 15
LENGTH : 30 base pairs
TYPE : nucleic acid

STRANDEDNESS : single
TOPOLOGY : linear
MOLECULE TYPE : other nucleic acid (synthetic DNA)

SEQUENCE DESCRIPTION:

AAACCCGGGA ATATACTAGA GATTTTTAAC 30

SEQ ID NO : 16
LENGTH : 27 base pairs
TYPE : nucleic acid
STRANDEDNESS : single
TOPOLOGY : linear
MOLECULE TYPE : other nucleic acid (synthetic DNA)

SEQUENCE DESCRIPTION:

AAAGAATTCA AGCTTGAGCT CCTCGAG 27

SEQ ID NO : 17
LENGTH : 36 base pairs
TYPE : nucleic acid
STRANDEDNESS : single
TOPOLOGY : linear
MOLECULE TYPE : other nucleic acid (synthetic DNA)

SEQUENCE DESCRIPTION:

TAGCTGCAGG CTCGTCTCAT CATGAGACGC ACTATG 36

SEQ ID NO : 18
LENGTH : 36 base pairs
TYPE : nucleic acid
STRANDEDNESS : single
TOPOLOGY : linear
MOLECULE TYPE : other nucleic acid (synthetic DNA)

SEQUENCE DESCRIPTION:

AAAAGATCTT TAAGACACAC GGGATAGGAC ACCCCT 36

SEQ ID NO : 19
LENGTH : 30 base pairs
TYPE : nucleic acid
STRANDEDNESS : single
TOPOLOGY : linear
MOLECULE TYPE : other nucleic acid (synthetic DNA)

SEQUENCE DESCRIPTION:

AAAAGATCTG TGTCTTCGTG CACAACCGTG 30

SEQ ID NO : 20

LENGTH : 33 base pairs

TYPE : nucleic acid

STRANDEDNESS : single

TOPOLOGY : linear

MOLECULE TYPE : other nucleic acid (synthetic DNA)

SEQUENCE DESCRIPTION:

AAACTCGAGC TATTTCTTTG TTTTTTTGAG CAC 33

## Claims

1. A modified protein comprising an amino acid sequence wherein at least one cysteine residue is changed to an amino acid residue to which a lipid cannot bind in comparison with the amino acid sequence of the original protein.

2. The modified protein of claim 1 comprising an amino acid sequence wherein the cysteine residue closest to the amino-terminus is changed to an amino acid residue to which a lipid cannot bind in comparison with the amino acid sequence of the original protein.

3. The modified protein of claims 1 or 2 wherein said amino acid residue is selected from a group consisting of alanine, glutamic acid and serine residues.

4. The modified protein of any one of claims 1-3 wherein the original protein is an antigenic protein derived from *Treponema pallidum.*

5. The modified protein of claim 4 wherein the original protein is an antigenic protein from *Treponema pallidum* which has a molecular weight of about 47 kDa or about 17 kDa.

6. The modified protein of claim 5 comprising the amino acid sequence shown as SEQ ID NO: 1.

7. The modified protein of claim 5 comprising the amino acid sequence shown as SEQ ID NO: 2.

8. The modified protein of any one of claims 1-7 which is a fusion protein obtainable by linking two or more antigenic proteins from a microorganism or microorganisms.

9. The modified protein of claim 8 wherein said antigenic protein derived from a microorganism or microorganisms is a fusion protein comprising a *Treponema pallidum*-derived antigenic protein which has a molecular weight of about 47 kDa and/or a *Treponema pallidum*-derived antigenic protein which has a molecular weight of about 17 kDa.

10. The modified protein of claim 8 or 9 comprising the amino acid sequence shown as SEQ ID NO: 4.

11. DNA which encodes the modified protein of any one of claims 1-10, or complementary DNA thereto.

12. The DNA of claim 11 which comprises the nucleotide sequence shown as SEQ ID NO: 5, 6 or 8.

13. A recombinant vector containing the DNA of claim 11 or 12.

14. A transformant containing the recombinant vector of claim 13.

15. The transformant of claim 14 which has been deposited as FERM BP-5641, FERM BP-5642 or FERM BP-5763.

16. A process for producing a modified protein, comprising the steps of culturing a transformant into which a gene encoding the modified protein of any one of claims 1-10 is introduced, producing and accumulating the modified protein in the culture, and collecting the culture.

17. The process for producing a modified protein of claim 16, further comprising the steps of adding ammonium sulfate to the culture supernatant of the collected culture, centrifuging the mixture to obtain a precipitate, and dissolving the precipitate in an aqueous solution containing a surfactant.

18. A method for assaying an anti-*Treponema pallidum* antibody wherein the modified protein of any one of claims 4-10 is used as an antigen.

19. A reagent for assaying an anti*-Treponema pallidum* antibody, comprising the modified protein of any one of claims 4-10 as an antigen.

20. A diagnostic reagent for syphilis infection, comprising the modified protein of any one of claims 4-10 as an active ingredient.

21. A process for producing an anti-*Treponema pallidum* antibody wherein the modified protein of any one of claims 4-10 is used as an antigen.
